# EUROPEAN PATENT SPECIFICATION

(11) **EP 2 140 892 B1**
(45) Date of publication and mention of the grant of the patent: **31.10.2012**
(21) Application number: 09251682.2
(22) Date of filing: 30.06.2009
(51) Int. Cl.: A61M 5/142

(54) **Volumetric micropump**
Volumetrische Mikropumpe
Micropompe volumétrique

(30) Priority: 30.06.2008 US 76845
(43) Date of publication of application: 06.01.2010
(73) Proprietor: Animas Corporation, West Chester, PA 19380 (US)
(72) Inventor: O'Connor, Sean, West Chester, PA 19380 (US)
(74) Representative: Tunstall, Christopher Stephen

(56) References cited:
- WO-A-00/00241
- WO-A-2006/089958
- WO-A-2006/113408
- WO-A-2007/074363
- WO-A-2008/024808
- CH-A5- 688 224

## Description

### FIELD OF THE INVENTION

The present invention relates, in general, to drug delivery systems and, more particularly, to a communications system for a drug delivery device that may be remotely controlled. The present invention also relates to methods of assembling such a drug delivery device in a manner that improves reliability, accuracy and drug delivery in the device.

### BACKGROUND OF THE INVENTION

Diabetes mellitus is a chronic metabolic disorder caused by an inability of the pancreas to produce sufficient amounts of the hormone insulin so that the metabolism is unable to provide for the proper absorption of sugar and starch. This failure leads to hyperglycemia, i.e. the presence of an excessive amount of glucose within the blood plasma. Persistent hyperglycemia causes a variety of serious symptoms and life threatening long term complications such as dehydration, ketoacidosis, diabetic coma, cardiovascular diseases, chronic renal failure, retinal damage and nerve damages with the risk of amputation of extremities. Because healing is not yet possible, a permanent therapy is necessary which provides constant glycemic control in order to always maintain the level of blood glucose within normal limits. Such glycemic control is achieved by regularly supplying external insulin to the body of the patient to thereby reduce the elevated levels of blood glucose.

External insulin was commonly administered by means of multiple, daily injections of a mixture of rapid and intermediate acting insulin via a hypodermic syringe. While this treatment does not require the frequent estimation of blood glucose, it has been found that the degree of glycemic control achievable in this way is suboptimal because the delivery is unlike physiological insulin production, according to which insulin enters the bloodstream at a lower rate and over a more extended period of time. Improved glycemic control may be achieved by the so-called intensive insulinotherapy which is based on multiple daily injections, including one or two injections per day of long acting insulin for providing basal insulin and additional injections of rapidly acting insulin before each meal in an amount proportional to the size of the meal. Although traditional syringes have at least partly been replaced by insulin pens, the frequent injections are nevertheless very inconvenient for the patient, particularly those who are incapable of reliably self-administering injections.

Substantial improvements in diabetes therapy have been achieved by the development of the insulin infusion pump, relieving the patient of the need syringes or insulin pens and the administration of multiple, daily injections. The insulin pump allows for the delivery of insulin in a manner that bears greater similarity to the naturally occurring physiological processes and can be controlled to follow standard or individually modified protocols to give the patient better glycemic control.

Infusion pumps can be constructed as an implantable device for subcutaneous arrangement or can be constructed as an external device with an infusion set for subcutaneous infusion to the patient via the transcutaneous insertion of a catheter or cannula. External infusion pumps are mounted on clothing, hidden beneath or inside clothing, or mounted on the body and are generally controlled via a user interface built-in to the device. WO 2006/113408 A. WO 2008/024808 A and WO 00/00241 A disclose pumps for fluid delivery.

Regardless of the type of infusion pump, blood glucose monitoring is required to achieve acceptable glycemic control. For example, delivery of suitable amounts of insulin by the insulin pump requires that the patient frequently determines his or her blood glucose level and manually input this value into a user interface for the external pumps, which then calculates a suitable modification to the default or currently in-use insulin delivery protocol, i.e. dosage and timing, and subsequently communicates with the insulin pump to adjust its operation accordingly. The determination of blood glucose concentration is typically performed by means of a measuring device such as a hand-held electronic meter which receives blood samples via enzyme-based test strips and calculates the blood glucose value based on the enzymatic reaction.

Since the blood glucose meter is an important part of an effective glycemic control treatment program, integrating the measuring aspects of the meter into an external pump or the remote of a pump is desirable. Integration eliminates the need for the patient to carry a separate meter device, it offers added convenience and safety advantages by eliminating the manual input of the glucose readings, and may reduce instances of incorrect drug dosaging resulting inaccurate data entry.

### Brief Description of the Figures

The accompanying drawings, which are incorporated herein and constitute part of this specification, illustrate presently preferred embodiments of the invention, and, together with the general description given above and the detailed description given below, serve to explain features of the invention (wherein like numerals represent like elements), of which:

Figures 1A-1C are cross-sectional views of a pump engine . Figure 1A illustrates the entire pump engine, while Figures 1B and 1C illustrate a portion of the pump engine during a pump cycle.

Figures 2A-2C are cross-sectional views of a stepped piston, which can be used in embodiments of the present invention and the pump engine illustrated in Figures 1A-1C.

Figure 3 is a cross-sectional view of a pump engine with a stepped piston, according to an embodiment described and illustrated herein.

Figures 4A-4C are cross-sectional views of a pump engine, according to an embodiment described and illustrated herein. Figure 4A illustrates the pump engine at rest, while Figures 4B and 4C illustrate the pump engine during a pump cycle.

Figures 5A-5C are perspective and cross-sectional views of a pump engine, . The pump engine has minimal dead volume, and creates a continuous flow path at its full stroke position.

Figure 6 illustrates a pump engine, with minimal dead volume, coupled to a reservoir and infusion set .

Figures 7A-7B are perspective views of a pump engine with actuator, according to an embodiment described and illustrated herein.

Figures 8A-8E are perspective and cross sectional views of an outlet check valve .

Figures 9A-9B are perspective views that illustrate a method for making a support/elastic membrane assembly as illustrated in Figure 8D .

Figures 10A-10B are perspective and cross sectional views of a check valve . The check valve can be used as an inlet check valve, or an outlet check valve.

Figures 11A-11C are perspective and plan views of a mechanically activated valve . The mechanically activated valve is typically placed inside a pump chamber, and can be used as an outlet valve in any of the pump engines described and illustrated herein.

Figures 12A-12B are perspective and cross sectional views of a check valve, . The check valve can be placed between a pump chamber and a reservoir, or between a pump chamber and an infusion set. The check valve can be used with any of the pump engines described and illustrated herein.

Figure 13 is a cross sectional view of a pump engine . The pump engine is typically placed between a reservoir and an infusion set.

Figure 14 is a cross sectional view of a pump engine . The pump engine is typically placed between a reservoir and an infusion set.

Figure 15 is a perspective view of a valved accumulation chamber . The valved accumulation chamber can be placed between a pump chamber and an infusion set, and prevents inadvertent delivery of fluid. The valved accumulation chamber can be used with any of the pump engines described and illustrated herein.

Figures 16A-16B are cross-sectional views of a dual chamber pump engine, according to an embodiment described and illustrated herein.

Figures 17A-17B are perspective and cross sectional views of a hydrophobic check valve . The hydrophobic check valve can be used to vent air during the filling of a reservoir, and to prevent air from flowing into a reservoir when liquids are drawn from the reservoir.

Figures 18A-18B are perspective and cross sectional views of a hydrophobic check valve. The hydrophobic check valve can be used to vent air during the filling of a reservoir, and to prevent air from flowing into a reservoir when liquids are drawn from the reservoir.

Figures 19A-19B are perspective and cross sectional views of a hydrophilic/hydrophobic check valve . The hydrophilic/hydrophobic check valve can be used to vent air during the filling of a reservoir, and to prevent air from flowing into a reservoir when liquids are drawn from the reservoir.

Figures 20A-20B are perspective views of reservoirs . The reservoirs eliminate undesirable air pockets while filling, and are particularly useful when incorporated in the pump engines and systems described and illustrated herein.

Figures 21A-21B are cross sectional and perspective views of a peristaltic fluid counter. The peristaltic fluid counter measures the volume of fluid that flows through it, and is particularly useful when incorporated into the pump engines and systems described and illustrated herein.

### Detailed Description of the Figures

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

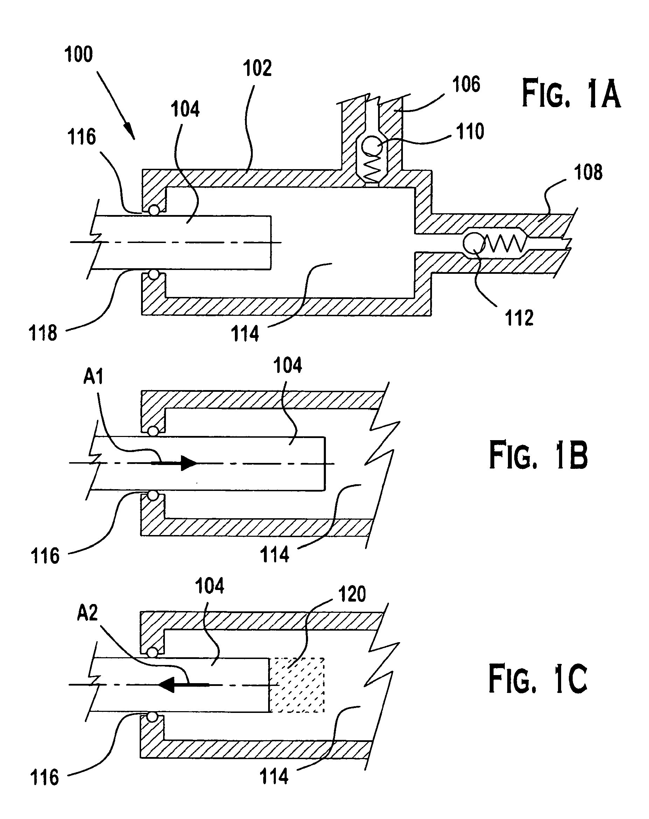

Figures 1A-1C are cross-sectional views of a pump engine 100 . Figure 1A illustrates the entire pump engine, while Figures 1B and 1C illustrate a portion of the pump engine during a pump cycle. Referring to Figure 1A, pump engine 100 comprises housing 102, piston 104, inlet 106, outlet 108, inlet check valve 110, outlet check valve 112, pump chamber 114, opening 116, and seal 118. Fluid flows into pump chamber 114 through inlet 106 and inlet check valve 110, while fluid flows out of pump chamber 114 through outlet 108 and outlet check valve 112. Inlet check valve 110 only allows flow into pump chamber 114, while outlet check valve 112 only allows flow out of pump chamber 114. Piston 104 enters pump chamber 114 through opening 116, and is sealed around its perimeter by seal 118. Piston 104 can move back and forth along its axis, while maintaining a hermetic seal between piston 104 and housing 102.

Housing 102 and piston 104 can be fabricated using a wide variety of materials, including, but not limited to, polymers, pure metals, metal alloys, ceramics, and silicon. Polymers include ABS, acrylic, fluoroplastics, polyamides, polyaryletherketones, PET, polycarbonate, polyethylene, PEEK, polypropylene, polystyrene, polyurethane, polyvinyl chloride, and polystyrene. Pure metals include titanium, platinum, or copper, while metal alloys include steel and nickel titanium (Nitinol). Seal 118 is typically made out of a polymer, such as natural or synthetic rubber, but can also be made out of metal, ceramic, or silicon. Inlet and outlet check valves 110 and 112 can be fabricated using polymers, metals, ceramics, and/or silicon, and frequently include a polymer component (such as a synthetic rubber ball or plug), and a metal component (such as a spring).

Figures 1B and 1C illustrate pump engine 100 during a pumping cycle. In Figure 1B, piston 104 has been moved away from the position illustrated in Figure 1A, in the direction indicated by arrow A1. As piston 104 moves in the direction indicated by arrow A1, the contents of pump chamber 114 increase in pressure, forcing inlet check valve 110 to close and outlet check valve 112 to open. As outlet check valve 112 opens, fluid flows from pump chamber 114, and through outlet check valve 112 and outlet 108. The volume displaced from pump chamber 114 is approximately equal to the volume displaced by piston 104 as piston 104 travels in the direction indicated by arrow A1. In Figure 1C, piston 104 travels back to its original position, as indicated by arrow A2. As piston 104 travels in the direction indicated by A2, the pressure in pump chamber 114 decreases, causing inlet check valve 110 to open and outlet check valve 112 to close. The decrease in pressure in pump chamber 114 causes fluid to flow through inlet 106 and inlet check valve 110 into pump chamber 114. The volume displaced from pump chamber 114 as piston 104 moves from the position illustrated in Figure 1A to the position illustrated in Figure 1B, and the volume that flows into pump chamber 114 as piston 104 travels from the position illustrated in Figure 1B to the position illustrated in Figure 1C, are illustrated by volume 120.

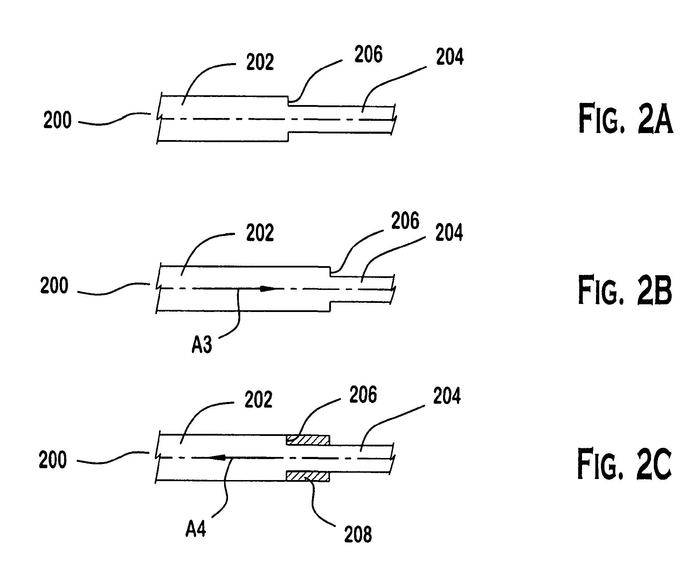

Figures 2A-2C are cross-sectional views of a stepped piston 200, which can be used in embodiments of the present invention, such as the pump engine illustrated in Figures 1A-1C. In Figure 2A, stepped piston 200 is in a home position, and includes first portion 202, second portion 204, and step 206. When used with a pump engine, such as that illustrated in Figures 1A-1C, first portion 202 and second portion 204 pass through walls in the housing, and occupy a portion of the pump chamber. Most of first portion 202, step 206, and second portion 204 are initially within the pump chamber, and remain within the pump chamber as stepped piston 200 moves back and forth. In Figure 2B, stepped piston 200 moves in the direction indicated by arrow A3, and step 206 comes to rest to the right of its original position. When stepped piston 200 moves in the direction indicated by arrow A3, it displaces fluid from the pump chamber in which it is mounted. In Figure 2C, stepped piston 200 moves in the direction indicated by arrow A4, back to the original position illustrated in Figure 2A. When stepped piston 200 moves from the position illustrated in Figure 2A to the position illustrated in Figure 2B, it displaces from the pump chamber a volume equal to volume 208. When stepped piston 200 moves from the position illustrated in Figure 2B to the position illustrated in Figure 2C, it draws into the pump chamber a volume equal to volume 208. There are advantages to using a stepped piston, as opposed to the piston illustrated in Figures 1A-1C. First, the stepped piston can be supported on both ends. This adds structural integrity to the piston. Second, a stepped piston allows finer resolution in terms of flow into and out of the pump chamber. For the same movement along its axis, a stepped piston will displace or draw a smaller volume of fluid.

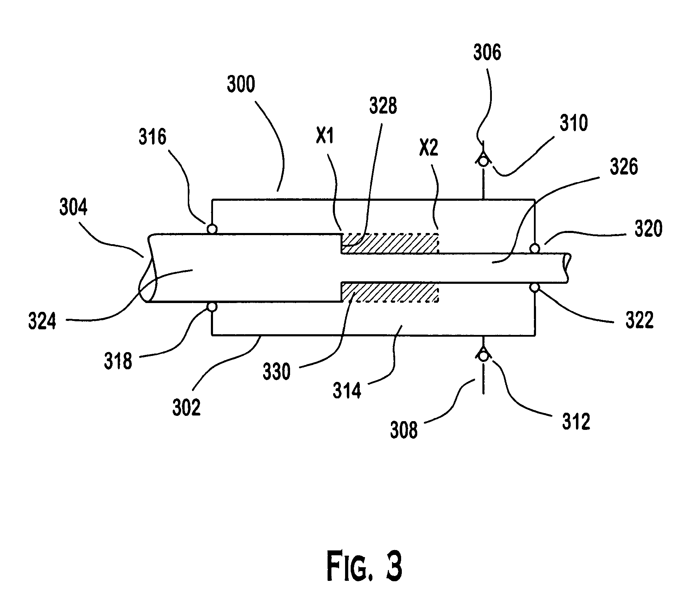

Figure 3 is a cross-sectional view of a pump engine 300 with a stepped piston 304, according to an embodiment described and illustrated herein. Referring to Figure 3, pump engine 300 comprises housing 302, stepped piston 304, inlet 306, outlet 308, inlet check valve 310, outlet check valve 312, pump chamber 314, first opening 316, first seal 318, second opening 320, and second seal 322. Stepped piston 304 includes first portion 324, second portion 326, and step 328. Fluid flows into pump chamber 314 through inlet 306 and inlet check valve 310, while fluid flows out of pump chamber 314 through outlet 308 and outlet check valve 312. Inlet check valve 310 only allows flow into pump chamber 314, while outlet check valve 312 only allows flow out of pump chamber 314. First portion 324 passes through first opening 316, and is sealed around its perimeter by first seal 318. Second portion 326 passes through second opening 320, and is sealed around its perimeter by second seal 322. Stepped piston 304 can move back and forth along its axis, while maintaining a hermetic seal between piston 304 and housing 302.

Housing 302 and piston 304 can be fabricated using a wide variety of materials, including, but not limited to, polymers, pure metals, metal alloys, ceramics, and silicon. Polymers include ABS, acrylic, fluoroplastics, polyamides, polyaryletherketones, PET, polycarbonate, polyethylene, PEEK, polypropylene, polystyrene, polyurethane, polyvinyl chloride, and polystyrene. Pure metals include titanium, platinum, or copper, while metal alloys include steel and nickel titanium (Nitinol). Seals 318 and 322 are typically made out of a polymer, such as natural or synthetic rubber, but can also be made out of metal, ceramic, or silicon. Inlet and outlet check valves 310 and 312 can be fabricated using polymers, metals, ceramics, and/or silicon, and frequently include a polymer component (such as a synthetic rubber ball or plug), and a metal component (such as a spring).

During a pump cycle, stepped piston 304 moves back and forth along its axis. For example, as step 328 is moved from position X1 to position X2, stepped piston 304 displaces a volume 330 from pump chamber 314. As step 328 is moved from position X2 to position X1, stepped piston 304 draws volume 330 into pump chamber 314. By cycling stepped piston back and forth, fluid is displaced from and drawn into pump chamber 314.

In micro pumps of the present invention, pump engines may be connected to reservoirs and infusion sets. In reference to Figures 1A-1C and Figure 3, a reservoir containing insulin can be connected to inlet 106 or inlet 306, and an infusion set can be connected to outlet 108 or 308. As the piston or stepped piston moves back and forth, insulin is displaced from then drawn into pump chambers 114 or 314. In this way, the pump engines illustrated in Figures 1 and 3 can be combined with reservoirs and infusion sets to provide micro pumps capable of delivering fluids such as insulin.

According to an embodiment described and illustrated herein, linear motors can be used to move stepped piston 304 back and forth. A preferred embodiment uses the Squiggle SQL Series Piezo Motor, which can be purchased from New Scale Technologies of Victor, New York. Squiggle SQL Series Piezo Motors are compact (approximately 1.55 mm x 1.55mm x 6 mm), are low cost, provide direct linear movement, and can move with sub-micron precision. The Squiggle SQL-1.5-6 can be used to build a low flow pump, where the diameter of first portion 324 is 1.8288mm (.0720 inches), the diameter of second portion 326 is 1.5875mm (.0625 inches), the stroke distance is 1.27mm (.050 inches), and the frequency is 1 Hz. The low flow pump delivers insulin at a maximum flow rate of 4.9 units/min (or 49 microliters/min) and a minimum flow rate of .010 units/hr (or . 1 microliters/hr), generating a pressure of 137.9kPa (20 psi) with a force of 9.1 grams. The Squiggle SQL-2.4-10 can be used to build a high flow pump, where the diameter of first portion 324 is 2.7788mm (.1094 inches), the diameter of second portion 326 is 1.5875mm (.0625 inches), the stroke distance is 2.032mm (.080 inches), and the frequency is 1 Hz. The high flow pump delivers insulin at a maximum flow rate of 49 units/min (or 490 microliters/min) and a minimum flow rate of .010 units/hr (or .1 microliters/hr), generating a pressure of 137.9kPa (20 psi) with a force of 57.4 grams. Although the use of linear motors to move pump pistons have been described in respect to the pump engine illustrated in Figure 3, they can be used in any of the embodiments described and illustrated herein, whenever linear motion is required.

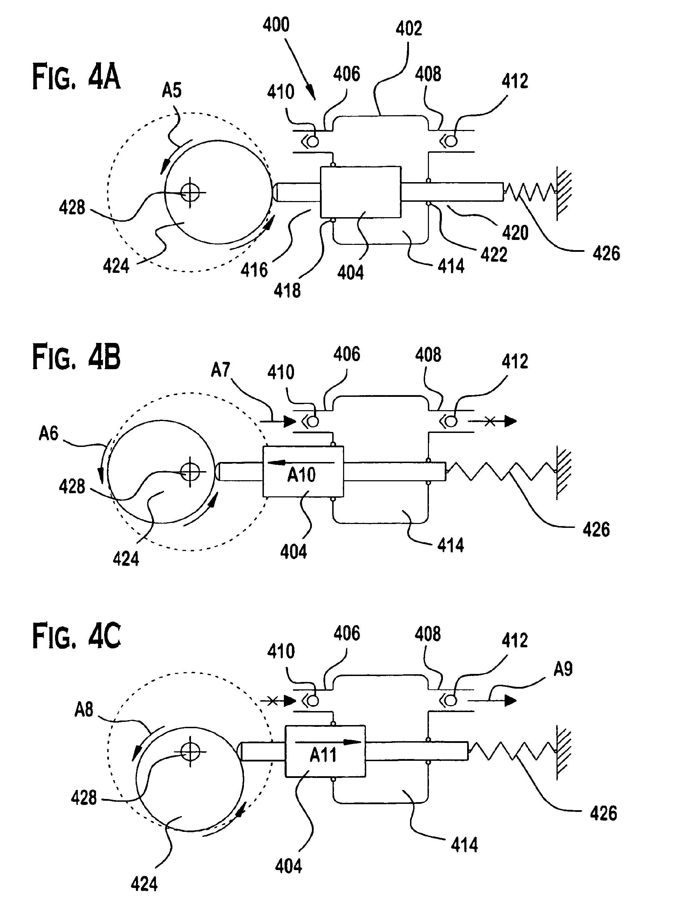

Figures 4A-4C are cross-sectional views of a pump engine 400, according to an embodiment described and illustrated herein. Figure 4A illustrates the pump engine at rest, while Figures 4B and 4C illustrate the pump engine during a pump cycle. Referring to Figure 4A, pump engine 400 comprises housing 402, stepped piston 404, inlet 406, outlet 408, inlet check valve 410, outlet check valve 412, pump chamber 414, first opening 116, first seal 418, second opening 420, second seal 422, cam 424, spring 426, and spindle 428. Fluid flows into pump chamber 414 through inlet 406 and inlet check valve 410, while fluid flows out of pump chamber 414 through outlet 408 and outlet check valve 412. Inlet check valve 410 only allows flow into pump chamber 414, while outlet check valve 412 only allows flow out of pump chamber 414. Stepped piston 404 passes through first opening 416, and is sealed around its perimeter by first seal 418. Stepped piston 404 also passes through second opening 420, and is sealed around its perimeter by second seal 422. Stepped piston 404 can move back and forth along its axis, while maintaining a hermetic seal between stepped piston 404 and housing 402. Cam 424 rotates about spindle 428, contacting and imparting linear motion to stepped piston 404. Spring 426 contacts stepped piston 404 at the opposite end, causing stepped piston to maintain contact with cam 424 as it rotates about spindle 428.

Housing 402, piston 404, cam 424, and spindle 428 can be fabricated using a wide variety of materials, including, but not limited to, polymers, pure metals, metal alloys, ceramics, and silicon. Polymers include ABS, acrylic, fluoroplastics, polyamides, polyaryletherketones, PET, polycarbonate, polyethylene, PEEK, polypropylene, polystyrene, polyurethane, polyvinyl chloride, and polystyrene. Pure metals include titanium, platinum, or copper, while metal alloys include steel and nickel titanium (Nitinol). Seals 418 and 422 are typically made out of a polymer, such as natural or synthetic rubber, but can also be made out of metal, ceramic, or silicon. Inlet and outlet check valves 410 and 412 can be fabricated using polymers, metals, ceramics, and/or silicon, and frequently include a polymer component (such as a synthetic rubber ball or plug), and a metal component (such as a spring).

Figures 4B and 4C illustrate pump engine 400 during a pumping cycle. In Figure 4B, stepped piston 404 has moved in the direction indicated by arrow A10. Stepped piston 404 moves in the direction indicated by arrow A10 due to force exerted by spring 426, and by the position of contact with cam 424. As cam 424 rotates about spindle 428, the position of contact between cam 424 and stepped piston 404 changes, allowing spring 426 to push more or less in the direction of arrow A10. As piston 404 moves in the direction indicated by arrow A10, the contents of pump chamber 414 decrease in pressure, forcing inlet check valve 410 to open and outlet check valve 412 to close. As inlet check valve 410 opens, fluid flows through inlet check valve 410 and inlet 406 and into pump chamber 414. The volume that flows into pump chamber 414 is approximately equal to the change in pump chamber volume occupied by stepped piston 404 as it travels in the direction indicated by arrow A10. Since stepped piston 404 is stepped, the volume it occupies in pump chamber 414 decreases as it moves in the direction indicated by arrow A10. In Figure 4C, stepped piston 404 travels in the direction indicated by arrow A11. As piston 404 travels in the direction indicated by A11, the pressure in pump chamber 414 increases, causing inlet check valve 410 to close and outlet check valve 412 to open. The increase in pressure in pump chamber 414 causes fluid to flow from pump chamber 414 and through outlet check valve 412 and outlet 408. The volume displaced from pump chamber 414 as stepped piston 404 moves from the position illustrated in Figure 4B to the position illustrated in Figure 4C is approximately equal to the increase in volume displaced by stepped piston 404 as it moves in the direction of arrow A11. In Figure 4C, stepped piston 404 moves in the direction indicated by arrow A11 due to a change in the point of contact between cam 424 and stepped piston 404 as cam 424 rotates about spindle 428. As cam 424 rotates about 428, the point of contact between cam 424 and stepped piston 404 moves along the axis of stepped piston 404 in the direction indicated by arrow A11.

As mentioned previously, in embodiments of the present invention, pump engines may be connected to reservoirs and infusion sets. In reference to Figures 4A-4C, a reservoir containing insulin can be connected to inlet 406, and an infusion set can be connected to outlet 408. As stepped piston 404 moves back and forth, insulin is drawn into then displaced from pump chamber 414. In this way, the pump engine illustrated in Figures 4A-4C can be combined with reservoirs and infusion sets to provide micro pumps capable of delivering fluids such as insulin.

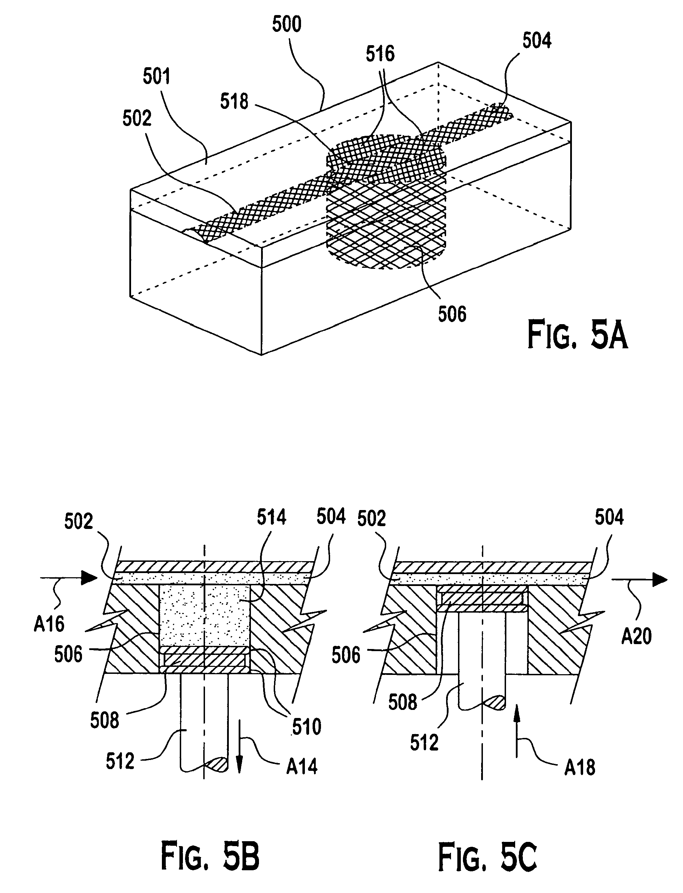

Figures 5A-5C are perspective and cross-sectional views of a pump engine 500 . Pump engine 500 has minimal dead volume, and creates a continuous flow path at its full stroke position. As illustrated in Figures 5A-5C, pump engine 500 comprises housing 501, inlet 502, outlet 504, pump chamber 506, piston 508, seals 510, and shaft 512. Shaft 512 is connected to piston 508, and moves piston 508 back and forth within pump chamber 506. Seals 510 are connected to piston 508, and form a seal between piston 508 and the inner wall of pump chamber 506. Fluid flows into pump chamber 506 through inlet 502, and flows out of pump chamber 506 through outlet 504. Inlet 502 and outlet 504 can include valves (not shown) to control flow. To start the pump cycle illustrated in Figures 5A and 5B, a valve on outlet 504 is closed and a valve on inlet 502 is opened. In Figure 5B, shaft 512, piston 508, and seals 510 are moved in the direction indicated by arrow A14, decreasing the pressure in pump chamber 506. As pressure in pump chamber 506 decreases, fluid 514 is drawn into pump chamber 506 through inlet 502. Once piston 508 reaches its maximum stroke, the valve on inlet 502 is closed, and the valve on outlet 504 is opened. Then, as illustrated in Figure 5C, piston 508 is moved in the direction of arrow A18, increasing the pressure in pump chamber 508, and causing flow of fluid 514 through outlet 504. Pump chamber 506 includes top surface 516 which makes contact with piston 508 when piston 508 is in the position illustrated in Figure 5C. This ensures full displacement of fluid 514 from pump chamber 506, with the exception of a small volume of fluid in connecting channel 518. Connecting channel 518 remains open, regardless of the position of piston 508, and allows connection between components connected to inlet 502 and outlet 504 (such as reservoirs and infusion sets), as long as inlet and outlet valves are open. This allows filling of components connected to inlet 502 with minimal pump chamber dead volume.

Housing 501, piston 508, shaft 512 can be fabricated using a wide variety of materials, including, but not limited to, polymers, pure metals, metal alloys, ceramics, and silicon. Polymers include ABS, acrylic, fluoroplastics, polyamides, polyaryletherketones, PET, polycarbonate, polyethylene, PEEK, polypropylene, polystyrene, polyurethane, polyvinyl chloride, and polystyrene. Pure metals include titanium, platinum, or copper, while metal alloys include steel and nickel titanium (Nitinol). Seals 510 are typically made out of a polymer, such as natural or synthetic rubber, but can also be made out of metal, ceramic, or silicon.

As mentioned previously, in embodiments of the present invention, pump engines may be connected to reservoirs and infusion sets. In reference to Figures 5A-5C, a reservoir containing insulin can be connected to inlet 502, and an infusion set can be connected to outlet 504. As 508 moves back and forth, insulin is drawn into then displaced from pump chamber 506. In this way, the pump engine illustrated in Figures 5A-5C can be combined with reservoirs and infusion sets to provide micro pumps capable of delivering fluids such as insulin.

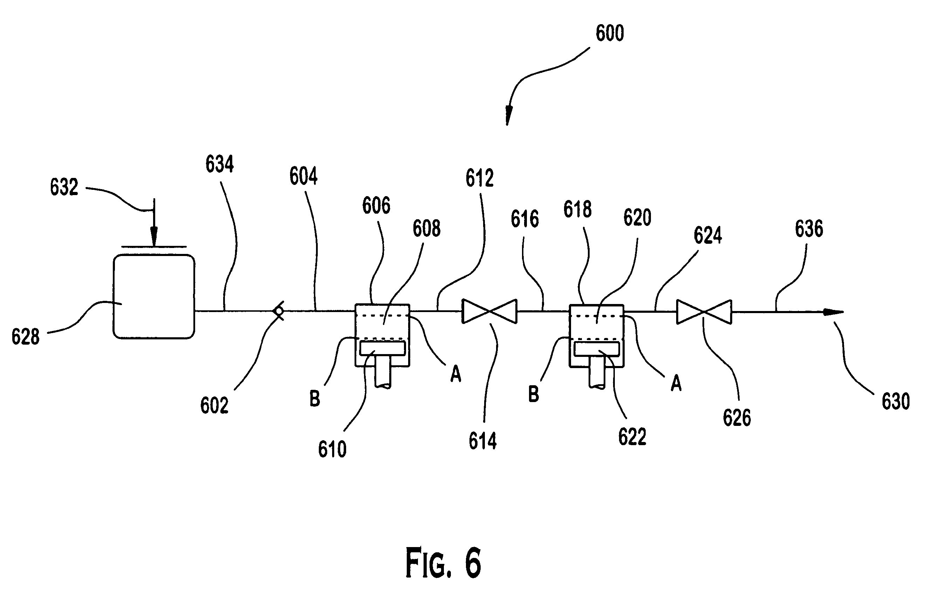

Figure 6 illustrates a pump engine, with minimal dead volume, coupled to a reservoir and infusion set. Pump engine 600 includes pump inlet 634, inlet check valve 602, first inlet channel 604, first housing 606, first pump chamber 608, first piston 610, first outlet channel 612, first valve 614, second inlet channel 616, second housing 618, second pump chamber 620, second piston 622, second outlet channel 624, second valve 626, and pump outlet 636. Reservoir 628 is connected to pump inlet 634, while infusion set 630 is connected to pump outlet 636. Positive displacement mechanism 632 pressurizes reservoir 628, ensuring complete flow from reservoir 628. Initially, second valve 626 is closed, first valve 614 is open, second piston 622 is in position A, and first piston 610 is in position A. A pre-filled reservoir 628 is connected to pump inlet 634, and pressure is applied by positive displacement mechanism 632. Next, second valve 626 remains closed, first valve 614 remains open, second piston 622 moves to position B, and first piston 610 moves to position B. This step fills first pump chamber 608 and second pump chamber 620 by drawing fluid from reservoir 628 and through pump inlet 634, inlet check valve 602, first inlet channel 604, first outlet channel 612, first valve 614, and second inlet channel 616 and is the point at which the pump cycle is subsequently repeated. Next, first valve 614 is closed, second valve 626 is opened, and second piston 622 is moved from position B to position A. This transfers fluid from second pump chamber 620 through second outlet channel 624, second valve 626, pump outlet 636, and into infusion set 630. Next, second valve 626 is closed, first valve 614 is opened, and first piston 612 is moved from position B to position A. This refills second pump chamber 620, and prepares first pump chamber 608 to be refilled. Fluid does not flow from first pump chamber 608 towards reservoir 628 because inlet check valve 602 does not allow flow in that direction. Finally, first valve 614 is closed and first piston 610 is moved from position A to position B, drawing fluid from reservoir 628, through pump inlet 634, inlet check valve 602, and first inlet channel 604, into first pump chamber 608. The pumping cycle is then repeated. The two-chamber, redundant pump engine described above is particularly advantageous because it prevents inadvertent free flow of fluid from reservoir 628 through infusion set 630.

Inlet check valve 602, first housing 606, first piston 610, first valve 614, second housing 618, second piston 622, and second valve 626 can be fabricated using a wide variety of materials, including, but not limited to, polymers, pure metals, metal alloys, ceramics, and silicon. Polymers include ABS, acrylic, fluoroplastics, polyamides, polyaryletherketones, PET, polycarbonate, polyethylene, PEEK, polypropylene, polystyrene, polyurethane, polyvinyl chloride, and polystyrene. Pure metals include titanium, platinum, or copper, while metal alloys include steel and nickel titanium (Nitinol).

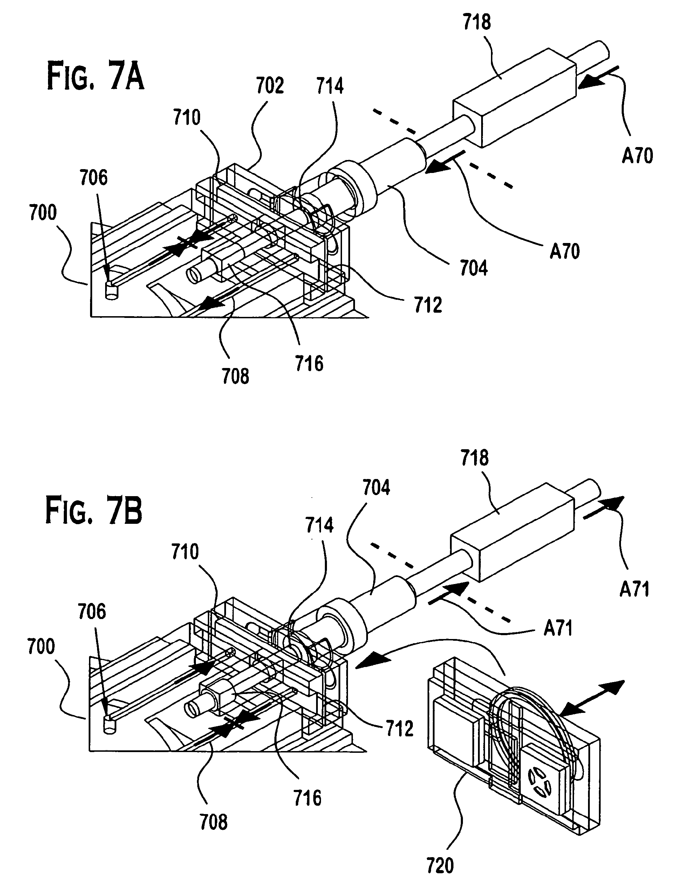

Figures 7A-7B are perspective views of a pump engine with actuator 700, according to an embodiment described and illustrated herein. Pump engine with actuator 700 comprises housing 702, stepped piston 704, inlet 706, outlet 708, inlet check valve 710, outlet check valve 712, pump chamber 714, spring 716, and actuator 718. Inlet 706 can be connected to a reservoir, while outlet 708 can be connected to an infusion set. Actuator 718 can be a linear motor, such as the Squiggle SQL Series Piezo Motor, mentioned previously. In figure 7A, actuator 718 moves in the direction indicated by arrows A70, forcing stepped piston 704 into pump chamber 714. As stepped piston 704 enters pump chamber 714, the pressure in pump chamber 714 builds, causing inlet check valve 710 to close and outlet check valve 712 to open. As outlet check valve 712 opens, fluid flows from pump chamber 714 through outlet check valve 712 and outlet 708. In Figure 7B, actuator 718 moves in the direction indicated by arrows A71, and spring 716 pushes stepped piston 704 away from pump chamber 714. As stepped piston 704 moves away from pump chamber 714, the pressure in pump chamber 714 drops, opening inlet check valve 710 and closing outlet check valve 712. Fluid is drawn through inlet 706 and inlet check valve 710 into pump chamber 714. The pump cycle illustrated in Figures 7A and 7B is then repeated. In Figure 7B, diaphragm pump engine 720 can be used in place of the stepped piston pump engine, in some embodiments.

Housing 702, stepped piston 704, inlet check valve 710, outlet check valve 712, spring 716, and diaphragm pump engine 720 can be fabricated using a wide variety of materials, including, but not limited to, polymers, pure metals, metal alloys, ceramics, and silicon. Polymers include ABS, acrylic, fluoroplastics, polyamides, polyaryletherketones, PET, polycarbonate, polyethylene, PEEK, polypropylene, polystyrene, polyurethane, polyvinyl chloride, and polystyrene. Pure metals include titanium, platinum, or copper, while metal alloys include steel and nickel titanium (Nitinol).

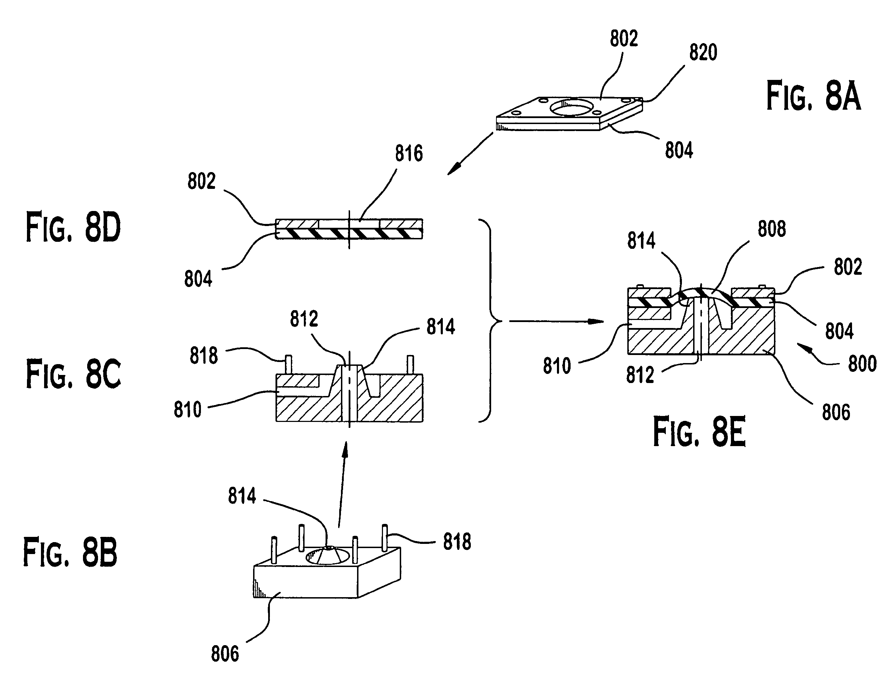

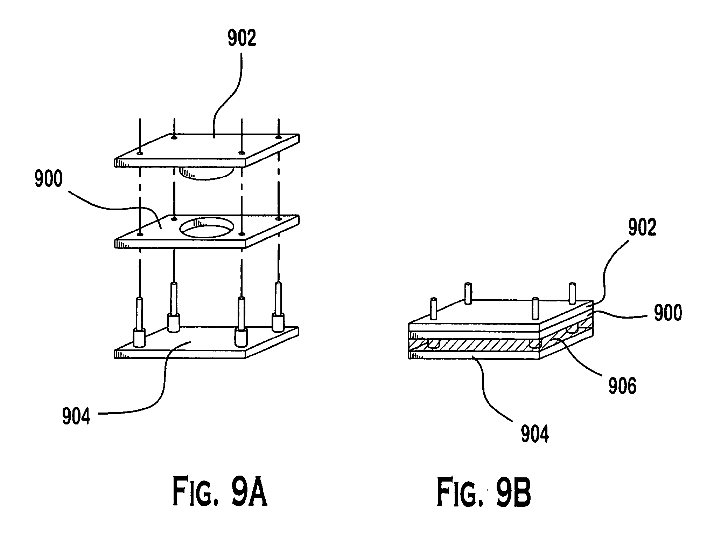

Figures 8A-8E are perspective and cross sectional views of an outlet check valve 800 . Outlet check valve 800 comprises support 802, elastic membrane 804, and valve block 806. Elastic membrane 804 includes sealing portion 808 and is connected to support 802, which includes opening 816 and alignment holes 820. Valve block 806 includes first channel 810, second channel 812, sealing surface 814, and alignment pins 818. Figure 8A is a perspective view of support 802 and elastic membrane 804. Elastic membrane 804 is connected to support 802, and is typically made out of a thin, flexible material, such as rubber. Support 802 is typically rigid, and can be made out of a thin rigid material, such as metal or plastic. Support 802 and elastic membrane 804 can be mechanically attached or fastened, or can be attached using adhesives. They can also be attached using insert molding, as will be described in respect to Figures 9A-9B. Support 802 includes opening 816, which allows elastic membrane 804 to flex back and forth during operation of outlet check valve 800. Figure 8B is a perspective view of valve block 806. Valve block 806 is typically made out of a rigid material, such as metal or plastic, and includes alignment pins 818, which aid in assembly of outlet check valve 800. Sealing surface 814 interacts with elastic membrane 804, forming a seal between elastic membrane 804 and valve block 806. Figure 8C is a cross sectional view of valve block 806, and illustrates first channel 810 and second channel 812. First channel 810 enters from the edge of valve block 806, and includes an annular space around the base of sealing surface 814. Second channel 812 connects sealing surface 814 with the bottom of valve block 806. Figure 8D is a cross sectional view of support 802 and elastic membrane 804, prior to assembly with valve block 806. Support 802, elastic membrane 804, and valve block 806 are concentrically aligned prior to assembly. Figure 8E is a cross sectional view of outlet check valve 800, once it has been assembled. Sealing portion 808 is in direct contact with sealing surface 814, and is stretched to provide sealing force against sealing surface 814. When pressure builds in first channel 810, sealing portion 808 is pushed up, disengaging sealing portion 808 from sealing surface 814, and allowing fluid to flow from first channel 810 to second channel 812. Conversely, when pressure builds in second channel 812, sealing portion 808 is pushed up, disengaging sealing portion 808 from sealing surface 814, and allowing fluid to flow from second channel 812 to first channel 810. As long as the pressure in first channel 810 or second channel 812 is greater than the pressure surrounding outlet check valve 800 and the force pushing sealing portion 808 up is greater than the tension pulling sealing portion 808 down, fluid can flow between first channel 810 and second channel 812 (in either direction). Outlet check valve 800 is particularly useful when incorporated in the pump engines and systems described previously. For example, outlet check valve 800 can be placed between a pump chamber and infusion set, allowing flow only when a positive pressure is created in the pump chamber. When a negative pressure (less than the pressure surrounding outlet check valve 800) is created in the pump chamber, sealing portion 808 pushes against sealing surface 814, preventing flow from the pump chamber to the infusion set, as is the case when the pump chamber is drawing fluid from a reservoir.

Figures 9A-9B are perspective views that illustrate a method for making a support/elastic membrane assembly as illustrated in Figure 8D . The method for making the support/elastic membrane assembly includes overmolding an elastomer directly onto a rigid support. This assembly method could be more economical, and provide a more consistent assembly than can be accomplished using mechanical or adhesive based assembly. In Figure 9A, support 900 is sandwiched between an upper mold cavity 902 and a lower mold cavity 904. In Figure 9B, thermoplastic or thermosetting elastomer is injected into a cavity 906 surrounding support 900. Once the elastomer has cooled or set, the support/elastic membrane assembly is removed from upper mold cavity 902 and lower mold cavity 904, and used in an outlet check valve, such as that illustrated in Figures 8A-8E.

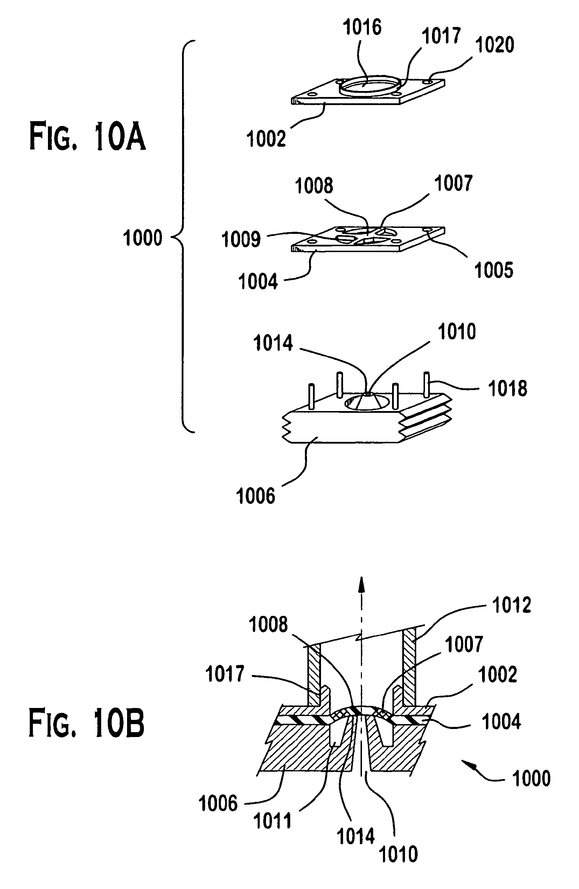

Figures 10A-10B are perspective and cross sectional views of a check valve 1000 . Check valve 1000 can be used as an inlet check valve, or an outlet check valve. Check valve 1000 comprises support 1002, elastic membrane 1004, and valve block 1006. Support 1002 includes opening 1016, collar 1017, and alignment holes 1020. Elastic membrane 1004 includes sealing portion 1008, ribs 1007, alignment holes 1005, and openings 1009. Valve block 1006 includes first channel 1010, annular region 1011, sealing surface 1014, and alignment pins 1018. Figure 10A is a perspective assembly view of support 1002, elastic membrane 1004, and valve block 1006. When check valve 1000 is assembled, elastic membrane 1004 is sandwiched between support 1002 and valve block 1006. Support 1002, elastic membrane 1004, and valve block 1006 can be mechanically attached or fastened, or can be attached using adhesives. They can also be attached using insert molding, as previously described in respect to Figures 9A-9B. Support 1002 includes opening 1016, which allows elastic membrane 1004 to flex back and forth during operation of check valve 1000. Opening 1016 also allows fluid to flow in or out of check valve 1000. Support 1002 includes collar 1017, which can be used to attach second channel 1012 to support 1002. Alignment holes 1020 are used in assembly, and assure registration between support 1002, elastic membrane 1004, and valve block 1006. Support 1002 is typically rigid, and can be made out of a thin rigid material, such as metal or plastic. Elastic membrane 1004 includes sealing portion 1008, ribs 1007, and openings 1009. Ribs 1007 connect sealing portion 1008 to the main body of elastic membrane 1004, allowing sealing portion 1008 to stretch back and forth as check valve 1000 opens and closes. Openings 1009 provide a flow path for fluid to flow between first channel 1010 and second channel 1012. Openings 1009 are aligned with annular region 1011, allowing fluid to flow to and from annular region 1011, first channel 1010, and second channel 1012. Elastic membrane 1004 is typically made out of a thin, flexible material, such as rubber. Valve block 1006 is typically made out of a rigid material, such as metal or plastic, and includes alignment pins 1018, which aid in assembly of check valve 1000. Sealing surface 1014 interacts with sealing portion 1008, forming a seal between elastic membrane 1004 and valve block 1006. Figure 10B is a cross sectional view of check valve 1000 and valve block 1006, and illustrates first channel 1010 and second channel 1012. First channel 1010 enters from the edge of valve block 1006, and is surrounded by annular region 1011 at the base of sealing surface 1014. Second channel 1012 connects to support 1002, and forms a fluidic pathway with first channel 1010 and annular region 1011. As illustrated in Figure 10B, sealing portion 1008 is in direct contact with sealing surface 1014, and is stretched to provide sealing force against sealing surface 1014. When pressure builds in first channel 1010, sealing portion 1008 is pushed up, disengaging sealing portion 1008 from sealing surface 1014, and allowing fluid to flow from first channel 1010 to annular region 1011, then through openings 1009 into second channel 1012. Alternatively, when pressure decreases in second channel 1012, sealing portion 1008 is pulled up, disengaging sealing portion 1008 from sealing surface 1014, and allowing fluid to flow from first channel 1010 to annular region 1011, then through openings 1009 into second channel 1012. As long as the pressure in first channel 1010 is greater than the pressure in second channel 1012, and the force pushing sealing portion 1008 up is greater than the tension pulling sealing portion 1008 down, fluid can flow between first channel 1010 and second channel 1012. Check valve 1000 is particularly useful when incorporated in the pump engines and systems described previously. For example, check valve 1000 can be placed between a pump chamber and infusion set, allowing flow only when a positive pressure is created in the pump chamber. When check valve 1000 is placed between a pump chamber and an infusion set, the infusion set is typically connected to second channel 1012 while the pump chamber is typically connected to first channel 1010. When a positive pressure (more than the pressure in the infusion set) is created in the pump chamber, sealing portion 1008 moves away from sealing surface 1014, allowing flow from the pump chamber to the infusion set. Alternatively, check valve 1000 can be placed between a pump chamber and a reservoir, with the reservoir typically connected to first channel 1010 and the pump chamber typically connected to second channel 1012. When a negative pressure (less than the pressure in the reservoir) is created in the pump chamber, sealing portion 1008 moves away from sealing surface 1014, allowing flow from the reservoir to the pump chamber.

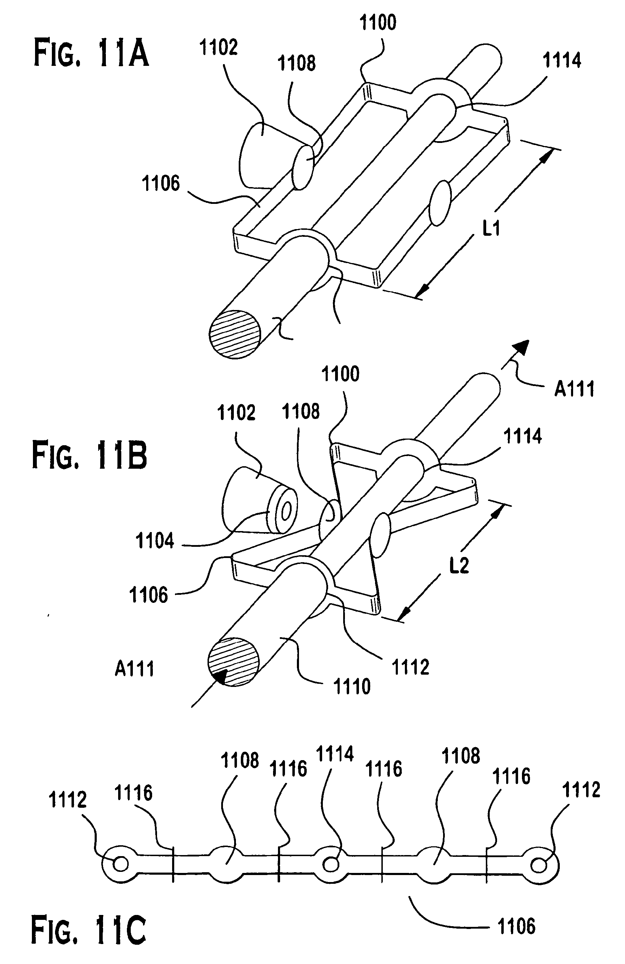

Figures 11A-11C are perspective and plan views of a mechanically activated valve 1100. Mechanically activated valve 1100 is typically placed inside a pump chamber, and can be used as an outlet valve in any of the pump engines described and illustrated herein. Mechanically activated valve 1100 comprises outlet channel 1102, flexible valve cover 1106, and piston 1110. Outlet channel 1102 includes sealing surface 1104 (which can be made out of an elastomer), and is typically connected to an infusion set. Piston 1110 can be either stepped or not stepped, and moves from its rest position (illustrated in Figure 11A), to its forward position (illustrated by arrows A111 in Figure 11B), and back to its rest position during a pump cycle. In its rest position, sealing portion 1108 of flexible valve cover 1106 is in contact with sealing surface 1104, preventing fluid from flowing through outlet channel 1102. As piston 1110 moves in the direction indicated by arrows A111, the distance L1 between first hole 1112 and second hole 1114 decreases, pulling sealing portion 1108 away from sealing surface 1104, allowing fluid to flow through outlet channel 1102. In Figure 11B, the distance between first hole 1112 and second hole 1114 (L2) is short enough to allow sealing portion 1108 to move away from sealing surface 1104. Due to mechanical fatigue, valve cover 1106 is usually fabricated using super elastic materials, such as Nitinol. As illustrated in Figure 11C, valve cover 1106 can be fabricated from a single sheet of Nitinol, with first holes 1112, second hole 1114, and sealing portions 1108. During fabrication, valve cover 1106 can be bent at bend locations 1116, and formed into the shape illustrated in Figure 11A. Although mechanically activated valve 1100 is actuated (while the check valves illustrated in Figures 8, 9, and 10 are not), mechanically activated valve 1100 can be actuated with the pump's piston, eliminating the need for an additional actuator.

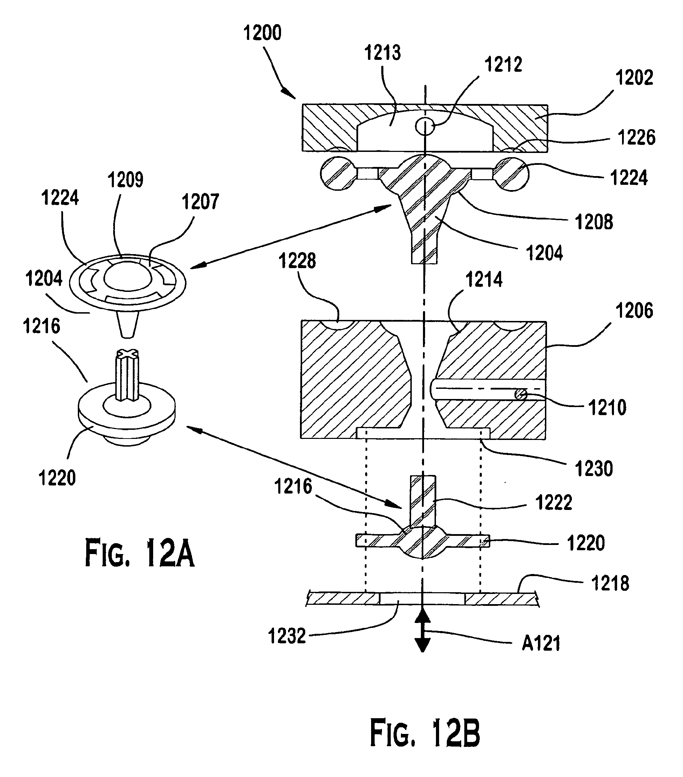

Figures 12A-12B are perspective and cross sectional views of a check valve 1200 . Check valve 1200 can be placed between a pump chamber and a reservoir, or between a pump chamber and an infusion set. Check valve 1200 can be used with any of the pump engines described and illustrated herein. Check valve 1200 can open or close due to differences in pressure across the valve inlet and outlets; it can also open or close due to external actuation. Check valve 1200 comprises top cover 1202, valve stem 1204, valve block 1206, internal actuator 1216, and bottom cover 1218. Top cover 1202, valve block 1206, and bottom cover 1218 are typically made out of a rigid material, such as metal or plastic, while valve stem 1204 and internal actuator 1216 are typically made out of an elastomer. Figure 12A is a perspective view of both valve stem 1204 and internal actuator 1216, while Figure 12B is a cross sectional assembly view of check valve 1200, prior to assembly. Top cover 1202 includes second channel 1212, sealing groove 1226, and upper chamber 1213. Upper chamber 1213 provides room for valve stem 1204, as valve stem 1204 moves up and down. Sealing groove 1226 mates with perimeter seal 1224, providing a hermetic seal between top cover 1202 and valve stem 1204. Second channel 1212 may be connected to a pump chamber, while second channel 1212 may also be connected to an infusion set. Valve stem 1204 includes ribs 1207, openings 1209, and perimeter seal 1224. Ribs 1207 connect the inner and outer portions of valve stem 1204, and allow the inner portion to move up or down. Openings 1209 allow fluid to pass through check valve 1200, when it is open. Perimeter seal forms a hermetic seal with top cover 1202 and valve block 1206. Valve stem 1204 also includes sealing portion 1208, which makes contact with sealing surface 1214 when check valve 1200 is closed. When check valve 1200 is open, sealing portion 1208 moves away from sealing surface 1214. Valve block 1206 includes first channel 1210, sealing surface 1214, sealing groove 1228, and sealing surface 1230. First channel 1210 can be connected to a reservoir or a pump chamber, and provides a conduit into the center of valve block 1206. Sealing surface 1214 contacts sealing portion 1208 when check valve 1200 is closed. Sealing groove 1228 makes contact with perimeter seal 1224, forming a hermetic seal between valve stem 1204 and valve block 1206. Sealing surface 1230 makes contact with flange 1220, forming a hermetic seal between valve block 1206 and internal actuator 1216. Internal actuator 1216 includes flange 1220 and shaft 1222. As mentioned previously, flange 1220 contacts sealing surface 1230, forming a hermetic seal between internal actuator 1216 and valve block 1206. Shaft 1222 extends into the center of valve block 1206, and can push valve stem 1204 and sealing portion 1208 away from sealing surface 1214, when the valve is opened. As indicated by arrow A121, internal actuator 1216, and shaft 1222, can move back and forth, opening and closing check valve 1200. Alternatively, a pressure differential across first channel 1210 and second channel 1212 can cause valve stem 1204 to move up or down, opening or closing the valve. Hence, check valve 1200 can be actively actuated (by pushing on internal actuator 1216), or check valve 1200 can be passively actuated (by relying on a pressure differential across first channel 1210 and second channel 1212). Bottom cover 1218 pushes flange 1220 against sealing surface 1230, and includes opening 1232 which allows access to internal actuator 1216 (so it can be pushed in to open the valve).

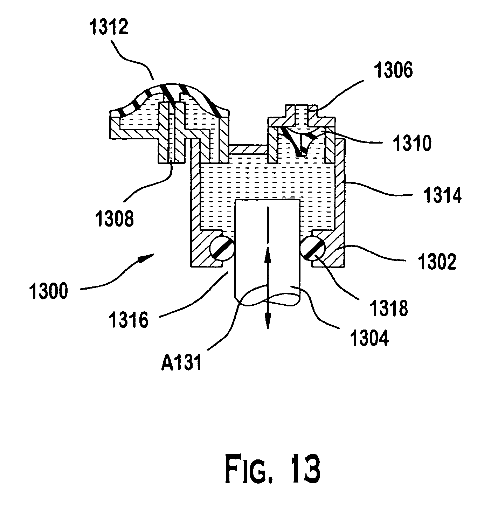

Figure 13 is a cross sectional view of pump engine 1300 . Pump engine 1300 is typically placed between a reservoir and an infusion set. Pump engine 1300 comprises housing 1302, piston 1304, inlet 1306, outlet 1308, inlet check valve 1310, outlet check valve 1312, pump chamber 1314, opening 1316, and seal 1318. Fluid flows into pump chamber 1314 through inlet 1306 and inlet check valve 1310, while fluid flows out of pump chamber 1314 through outlet 1308 and outlet check valve 1312. Inlet check valve 1310 only allows flow into pump chamber 1314, while outlet check valve 1312 only allows flow out of pump chamber 1314. Piston 1304 enters pump chamber 1314 through opening 1316, and is sealed around its perimeter by seal 1318. Piston 1304 can move back and forth along its axis (as indicated by arrow A131), while maintaining a hermetic seal between piston 1304 and housing 1302.

Housing 1302 and piston 1304 can be fabricated using a wide variety of materials, including, but not limited to, polymers, pure metals, metal alloys, ceramics, and silicon. Polymers include ABS, acrylic, fluoroplastics, polyamides, polyaryletherketones, PET, polycarbonate, polyethylene, PEEK, polypropylene, polystyrene, polyurethane, polyvinyl chloride, and polystyrene. Pure metals include titanium, platinum, or copper, while metal alloys include steel and nickel titanium (Nitinol). Seal 1318 is typically made out of a polymer, such as natural or synthetic rubber, but can also be made out of metal, ceramic, or silicon. Inlet and outlet check valves 1310 and 1312 can be fabricated using polymers (such as an elastomer), metals, and/or silicon.

As piston 1304 moves into pump chamber 1314, the contents of pump chamber 1314 increase in pressure, forcing inlet check valve 1310 to close and outlet check valve 1312 to open. As outlet check valve 1312 opens, fluid flows from pump chamber 1314, and through outlet check valve 1312 and outlet 1308. The volume displaced from pump chamber 1314 is approximately equal to the volume displaced by piston 1304 as piston 1304 travels into pump chamber 1314. As piston 1304 is drawn out of pump chamber 1314, the pressure in pump chamber 1314 decreases, causing inlet check valve 1310 to open and outlet check valve 1312 to close. The decrease in pressure in pump chamber 1314 causes fluid to flow through inlet 1306 and inlet check valve 1310 into pump chamber 1314. Inlet 1306 is typically connected to a reservoir, while outlet 1308 is typically connected to an infusion set. By reciprocating piston 1304 back and forth, fluid is drawn from a reservoir and transferred to an infusion set.

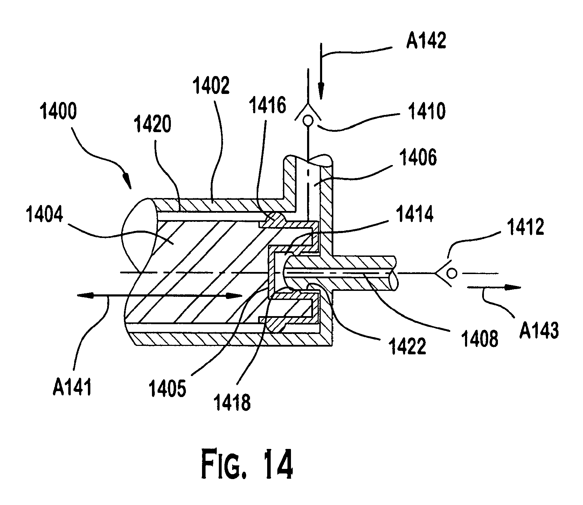

Figure 14 is a cross sectional view of pump engine 1400 . Pump engine 1400 is typically placed between a reservoir and an infusion set. Pump engine 1400 comprises housing 1402, piston 1404, piston cap 1405, inlet 1406, outlet 1408, inlet check valve 1410, outlet check valve 1412, pump chamber 1414, outer seal 1416, and inner seal 1418. Fluid flows into pump chamber 1414 through inlet 1406 and inlet check valve 1410, while fluid flows out of pump chamber 1414 through outlet 1408 and outlet check valve 1412. Inlet check valve 1410 only allows flow into pump chamber 1414, while outlet check valve 1412 only allows flow out of pump chamber 1414. Piston cap 1405 is mounted on the end of piston 1404, and includes outer seal 1416 and inner seal 1418. Outer seal 1416 contacts inner wall 1420 (forming a hermetic seal) while piston 1404 travels back and forth, as illustrated by arrow A141. Inner seal 1418 contacts outlet nib 1422 when piston 1404 is completely forward, preventing inadvertent leakage between inlet 1406 and outlet 1408 when the pump is off.

Housing 1402 and piston 1404 can be fabricated using a wide variety of materials, including, but not limited to, polymers, pure metals, metal alloys, ceramics, and silicon. Polymers include ABS, acrylic, fluoroplastics, polyamides, polyaryletherketones, PET, polycarbonate, polyethylene, PEEK, polypropylene, polystyrene, polyurethane, polyvinyl chloride, and polystyrene. Pure metals include titanium, platinum, or copper, while metal alloys include steel and nickel titanium (Nitinol). Piston cap 1405 is typically made out of a polymer, such as an elastomer, but can also be made out of metal, ceramic, or silicon. Inlet and outlet check valves 1410 and 1412 can be fabricated using polymers, metals, ceramics, and/or silicon, and frequently include a polymer component (such as a synthetic rubber ball or plug), and a metal component (such as a spring).

As piston 1404 moves into pump chamber 1414, the contents of pump chamber 1414 increase in pressure, forcing inlet check valve 1410 to close and outlet check valve 1412 to open. As outlet check valve 1412 opens, fluid flows from pump chamber 1414, and through outlet check valve 1412 and outlet 1408 (as indicated by arrow A143). The volume displaced from pump chamber 1414 is approximately equal to the volume displaced by piston 1404 as piston 1404 travels into pump chamber 1414. As piston 1404 is drawn back, the pressure in pump chamber 1414 decreases, causing inlet check valve 1410 to open and outlet check valve 1412 to close. The decrease in pressure in pump chamber 1414 causes fluid to flow through inlet 1406 and inlet check valve 1410 into pump chamber 1414 (as indicated by arrow A142). Inlet 1406 is typically connected to a reservoir, while outlet 1408 is typically connected to an infusion set. By reciprocating piston 1404 back and forth, fluid is drawn from a reservoir and transferred to an infusion set. Pump engine 1400 has the particular advantage that inner seal 1418 completely prevents flow when piston 1404 is completely forward, as illustrated in Figure 14.

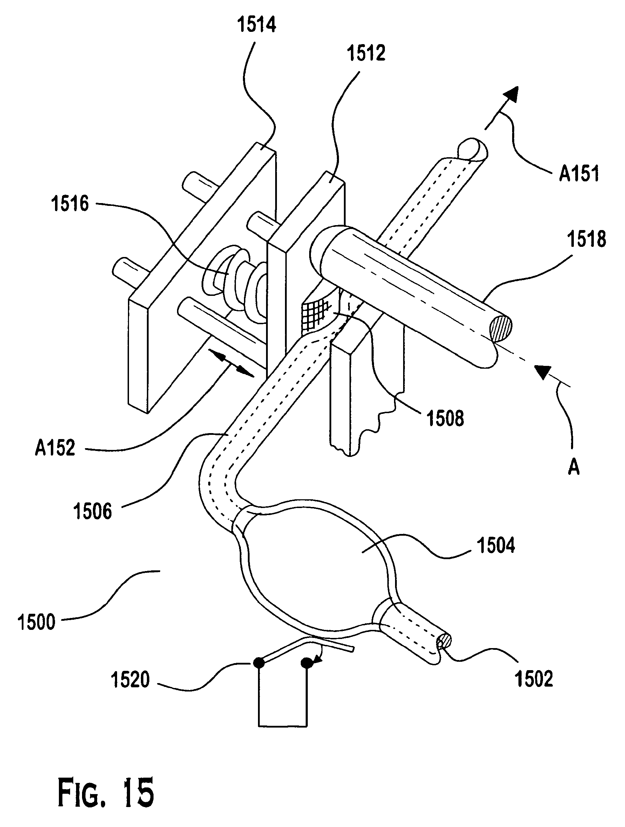

Figure 15 is a perspective view of a valved accumulation chamber 1500 . Valved accumulation chamber 1500 can be placed between a pump chamber and an infusion set, and prevents inadvertent delivery of fluid. Valved accumulation chamber 1500 can be used with any of the pump engines described and illustrated herein. Valved accumulation chamber 1500 opens at the end of a piston stroke, and is otherwise closed. Valved accumulation chamber 1500 comprises inlet 1502, compliant chamber 1504, outlet 1506, pinch point 1508, moveable plate 1512, base plate 1514, spring 1516, and sensor 1520. Inlet 1502 is typically connected to the outlet of a pump engine, while outlet 1506 is typically connected to an infusion set. Piston 1518, which is part of a pump engine, pushes against moveable plate 1512 at the end of its stroke, causing pinch point 1508 to loosen its grip on outlet 1506. When piston 1518 is not at full stroke, spring 1516 forces moveable plate 1512 and pinch point 1508 against outlet 1506, preventing flow through 1506. Fluid that leaves the pump engine prior to piston 1518 reaching full stroke accumulates in compliant chamber 1504. Once piston 1518 reaches full stroke, it pushes moveable plate 1512 and pinch point 1508 back, allowing fluid to flow from compliant chamber 1504 through outlet 1506, and into an infusion set, as indicated by arrow A151. Base plate 1514 is typically fixed, while moveable plate 1512 moves back and forth, as indicated by arrow A 152. Spring 1516 forces moveable plate 1512 into a normally closed position, preventing flow through outlet 1506 with pinch point 1508. Valved accumulation chamber 1500 prevents inadvertent flow by only allowing flow through outlet 1506 when piston 1518 is at full stroke. Sensor 1520 can be used to detect excess pressure in compliant chamber 1504, as might result when there is a flow blockage in the infusion set. When sensor 1520 detects excess pressure in compliant chamber 1504, warnings can be sent to the user, and the pump engine can be shut off.

Inlet 1502, outlet 1506, pinch point 1508, moveable plate 1512, base plate 1514, and spring 1516 can be fabricated using a wide variety of materials, including, but not limited to, polymers, pure metals, metal alloys, ceramics, and silicon. Polymers include ABS, acrylic, fluoroplastics, polyamides, polyaryletherketones, PET, polycarbonate, polyethylene, PEEK, polypropylene, polystyrene, polyurethane, polyvinyl chloride, and polystyrene. Pure metals include titanium, platinum, or copper, while metal alloys include steel and nickel titanium (Nitinol). Compliant chamber 1504 is typically made out of a polymer, such as an elastomer.

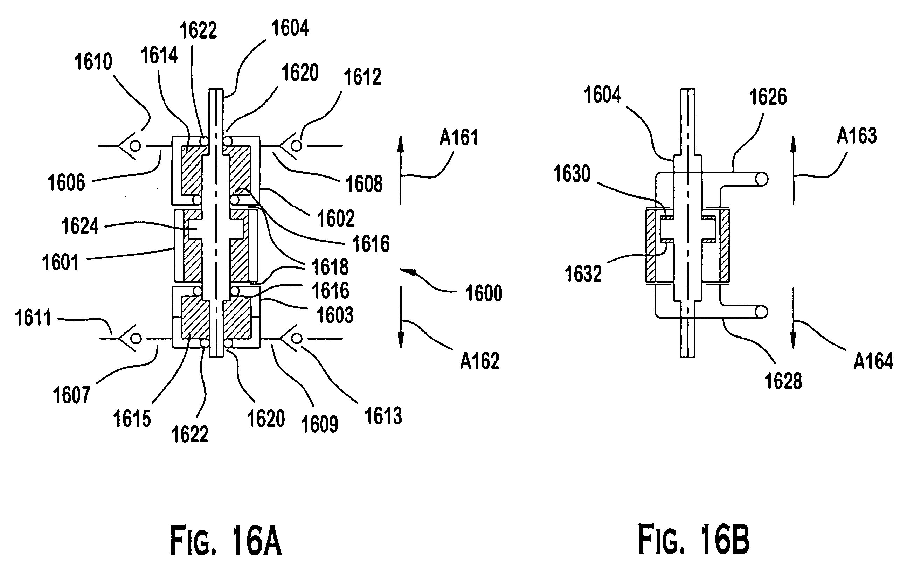

Figures 16A-16B are cross-sectional views of a dual chamber pump engine 1600 . Dual chamber pump engine 1600 comprises cylinder 1601, first housing 1602, second housing 1603, stepped piston 1604, first inlet 1606, second inlet 1607, first outlet 1608, second outlet 1609, first inlet check valve 1610, second inlet check valve 1611, first outlet check valve 1612, second outlet check valve 1613, first pump chamber 1614, second pump chamber 1615, first openings 1616, first seals 1618, second openings 1620, and second seals 1622. Inlet channels 1606 and 1607 may be connected to a reservoir, while outlet channels 1608 and 1609 may be connected to an infusion set. Stepped piston 1604 includes stepped regions in both the first and second pump chambers, and a piston stop 1624 in its middle. Piston stop 1624 limits the travel of stepped piston 1604 along its axis by interacting with the end surfaces of cylinder 1601. Fluid flows into pump chambers 1614 and 1615 through inlets 1606 and 1607 and inlet check valves 1610 and 1611, while fluid flows out of pump chambers 1614 and 1615 through outlets 1608 and 1609 and outlet check valves 1612 and 1613. Inlet check valves 1610 and 1611 only allow flow into pump chambers 1614 and 1615, while outlet check valves 1612 and 1613 only allow flow out of pump chambers 1614 and 1615. Stepped piston 1604 is sealed around its perimeter as it passes through openings 1616 and 1620 by seals 1618 and 1622. Stepped piston 1604 can move back and forth along its axis (as illustrated by arrows A161 and A162), while maintaining a hermetic seal between piston 1604 and housings 1602 and 1603.

Cylinder 1601, housings 1602 and 1603, and stepped piston 1604 can be fabricated using a wide variety of materials, including, but not limited to, polymers, pure metals, metal alloys, ceramics, and silicon. Polymers include ABS, acrylic, fluoroplastics, polyamides, polyaryletherketones, PET, polycarbonate, polyethylene, PEEK, polypropylene, polystyrene, polyurethane, polyvinyl chloride, and polystyrene. Pure metals include titanium, platinum, or copper, while metal alloys include steel and nickel titanium (Nitinol). Seals 1618 and 1622 are typically made out of a polymer, such as natural or synthetic rubber, but can also be made out of metal, ceramic, or silicon. Inlet and outlet check valves 1610, 1611, 1612 and 1613 can be fabricated using polymers, metals, ceramics, and/or silicon, and frequently include a polymer component (such as a synthetic rubber ball or plug), and a metal component (such as a spring).

During a pump cycle, stepped piston 1604 moves back and forth along its axis. For example, as stepped piston 1604 moves in the direction indicated by arrow A161, it pushes fluid from first pump chamber 1614, through first outlet 1608 and first outlet check valve 1612, into an infusion set. At the same time, stepped piston 1604 draws fluid from a reservoir, through second inlet 1607 and second inlet check valve 1611, and into second pump chamber 1615. Stepped piston 1604 then moves in the direction indicated by arrow A 162, drawing fluid from a reservoir, through first inlet 1606 and first inlet check valve 1610, into first pump chamber 1614. At the same time, it pushes fluid from second pump chamber 1615, through second outlet 1609 and second outlet check valve 1613, and into an infusion set.

Figure 16B illustrates a sensing mechanism for detecting maximum piston stroke. In this embodiment, stepped piston 1604 includes first conductive surface 1630 and second conductive surface 1632. As stepped piston 1604 moves in the direction indicated by arrow A163, and reaches its maximum stroke, first conductive surface 1630 contacts first circuit 1626. As first conductive surface 1630 contacts first circuit 1626, the circuit is completed, thus sensing the maximum stroke of stepped piston 1604 in the direction indicated by arrow A163. As stepped piston 1604 moves in the direction indicated by arrow A164, and reaches its maximum stroke, second conductive surface 1632 contacts second circuit 1628. As second conductive surface 1632 contacts second circuit 1628, the circuit is completed, thus sensing the maximum stroke of stepped piston 1604 in the direction indicated by arrow A 164. The sensing mechanism can be used to trigger actuation of stepped piston 1604. For example, a linear motor (as described previously) can be attached to one end of stepped piston 1604, while a spring is attached to the other end. The linear motor can be activated to move stepped piston 1604 in the direction indicated by arrow A163. As soon as the maximum stroke is reached, first circuit 1626 is completed, and the linear motor is turned off. The spring (which was compressed as stepped piston 1604 moved in the direction indicated by arrow A163) decompresses, pushing stepped piston 1604 in the direction indicated by arrow A164. As soon as the stepped piston reaches its maximum stroke, second circuit 1628 is completed, and the linear motor is turned back on, repeating the cycle.

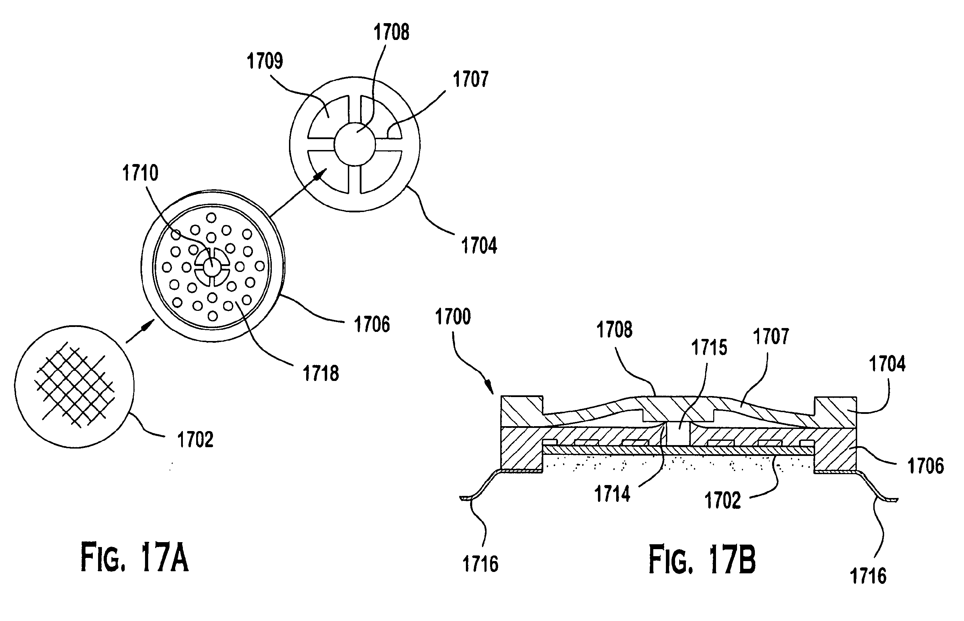

Figures 17A-17B are perspective and cross sectional views of a hydrophobic check valve 1700. Hydrophobic check valve 1700 can be used to vent air during the filling of a reservoir, and to prevent air from flowing into a reservoir when liquids are drawn from the reservoir. Hydrophobic check valve 1700 comprises hydrophobic membrane 1702, elastic membrane 1704, and valve block 1706. Hydrophobic membrane 1702 allows air to pass, but blocks water and aqueous solutions. Hydrophobic membranes can be made out of a variety of materials, including Nylon, fluoropolymers, and polypropylene. Elastic membrane 1704 includes sealing portion 1708, ribs 1707, and openings 1709. Elastic membrane 1704 can be made out of a variety of materials, but is often made out of an elastomer. Ribs 1707 allow sealing portion 1708 to stretch back and forth, as it seals and unseals against sealing surface 1714. Openings 1709 allow air to escape when hydrophobic check valve 1700 opens. Valve block 1706 includes inlet 1710, outlet 1711, sealing surface 1714, and bumps 1718. Bumps 1718 provide a gap between hydrophobic membrane 1702 and valve block 1706, allowing air to flow through hydrophobic membrane 1702 and into inlet 1710. Sealing surface 1714 surrounds outlet 1711, and forms a seal with sealing portion 1708 when the valve is closed. When hydrophobic check valve 1700 is assembled, elastic membrane 1704 is hermetically sealed at its edges to valve block 1706. In addition, hydrophobic membrane 1702 is hermetically sealed at its edges to the other side of valve block 1706. The outer edge of valve block 1706 can be hermetically attached to reservoir 1716, as shown in Figure 17B. Valve block 1706 is typically rigid, and can be made out a variety of materials, such as metal or plastic. Sealing portion 1708 is in direct contact with sealing surface 1714, and is stretched to provide sealing force against sealing surface 1714. When pressure builds in reservoir 1716, sealing portion 1708 is pushed up, disengaging sealing portion 1708 from sealing surface 1714, and allowing air to flow through hydrophobic membrane 1702 and valve block 1706. Alternatively, when pressure decreases in reservoir 1716, sealing portion 1708 is pushed against sealing surface 1714, preventing air from flowing into reservoir 1716. As long as atmospheric pressure is greater than or equal to the pressure in reservoir 1716, sealing portion 1708 will seal against sealing surface 1714, and prevent air from flowing through hydrophobic check valve 1700 into reservoir 1716. If the pressure in reservoir 1716 is greater than the sum of atmospheric pressure plus the elastic tension pulling sealing portion 1708 down, air will flow from reservoir 1716 and through hydrophobic check valve 1700. Hydrophobic check valve 1700 is particularly useful when incorporated in the pump engines and systems described and illustrated herein. For example, hydrophobic check valve 1700 can be attached to a reservoir, allowing air to escape when the reservoir is being filled, but preventing air from being drawn into the reservoir as fluid passes from the reservoir to the pump engine.

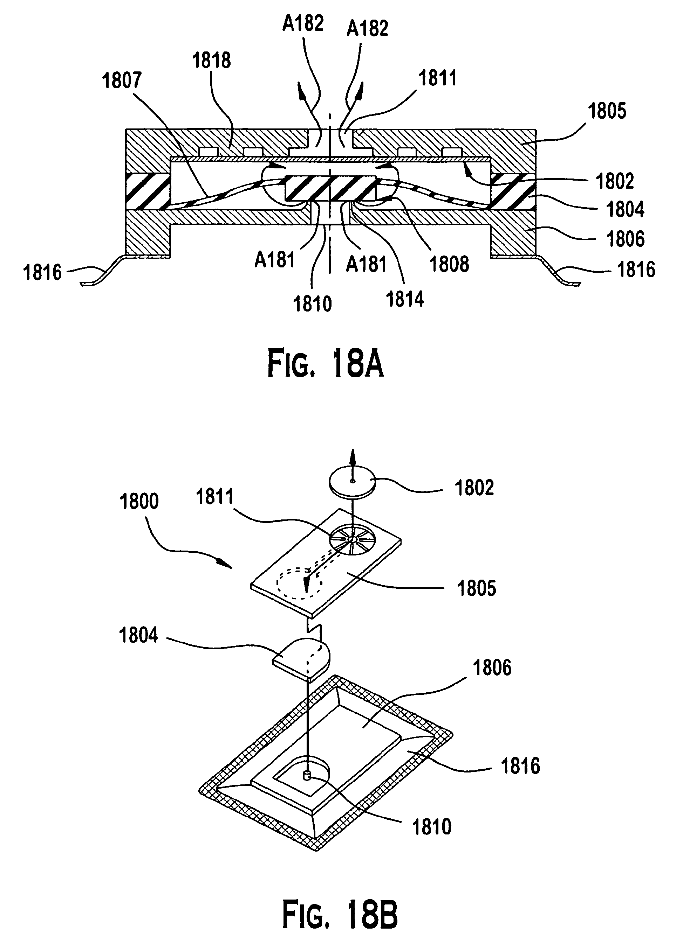

Figures 18A-18B are perspective and cross sectional views of a hydrophobic check valve 1800. Hydrophobic check valve 1800 can be used to vent air during the filling of a reservoir, and to prevent air from flowing into a reservoir when liquids are drawn from the reservoir. Hydrophobic check valve 1800 prevents direct contact, in the reservoir, between a hydrophobic membrane and the contents of the reservoir. This is particularly beneficial when the reservoir contains pharmaceutical solutions, such as insulin, since aggregates can form when pharmaceutical solutions are in direct contact with hydrophobic surfaces. Hydrophobic check valve 1800 comprises hydrophobic membrane 1802, elastic membrane 1804, valve block 1806, and top cover 1805. Hydrophobic membrane 1802 allows air to pass, but blocks water and aqueous solutions. Hydrophobic membranes can be made out of a variety of materials, including Nylon, fluoropolymers, and polypropylene. Elastic membrane 1804 includes sealing portion 1808, ribs 1807, and openings (not shown). Elastic membrane 1804 can be made out of a variety of materials, but is often made out of an elastomer. Ribs 1807 allow sealing portion 1808 to stretch back and forth, as it seals and unseals against sealing surface 1814. The openings (not shown) in elastic membrane 1804 allow air and liquid to escape when hydrophobic check valve 1800 opens. Top cover 1805 is typically made out of a rigid material, such as plastic or metal, and includes outlet 1811 and bumps 1818. Bumps 1818 provide a gap between hydrophobic membrane 1802 and top cover 1805, allowing air to flow through hydrophobic membrane 1802 and through outlet 1811. Valve block 1806 includes inlet 1810, and sealing surface 1814. Sealing surface 1814 surrounds inlet 1810, and forms a seal with sealing portion 1808 when the valve is closed. When hydrophobic check valve 1800 is assembled, elastic membrane 1804 is hermetically sealed at its edges to valve block 1806 and top cover 1805. In addition, hydrophobic membrane 1802 is hermetically sealed at its edges to the inside surface of top cover 1805. The outer edge of valve block 1806 can be hermetically attached to reservoir 1816, as shown in Figures 18A and 18B. Valve block 1806 is typically rigid, and can be made out a variety of materials, such as metal or plastic. Sealing portion 1808 is in direct contact with sealing surface 1814, and is stretched to provide sealing force against sealing surface 1814. When pressure builds in reservoir 1816, sealing portion 1808 is pushed up, disengaging sealing portion 1808 from sealing surface 1814, and allowing air and liquid to flow through valve block 1806 and elastic membrane 1804, as illustrated by arrows A181 and A182. Alternatively, when pressure decreases in reservoir 1816, sealing portion 1808 is pushed against sealing surface 1814, preventing air from flowing into reservoir 1816. As long as atmospheric pressure is greater than or equal to the pressure in reservoir 1816, sealing portion 1808 will seal against sealing surface 1814, and prevent air from flowing through hydrophobic check valve 1800 into reservoir 1816. If the pressure in reservoir 1816 is greater than the sum of atmospheric pressure plus the elastic tension pulling sealing portion 1808 down, air and liquid will flow from reservoir 1816 through valve block 1806 and elastic membrane 1804. Air will continue to pass through hydrophobic membrane 1802, but liquid will not. Hydrophobic check valve 1800 is particularly useful when incorporated in the pump engines and systems described and illustrated herein. For example, hydrophobic check valve 1800 can be attached to a reservoir, allowing air to escape when the reservoir is being filled, but preventing air from being drawn into the reservoir as fluid passes from the reservoir to the pump engine. Figure 18B illustrates a slightly different version of hydrophobic check valve 1800. In this version, hydrophobic membrane 1802 and elastic membrane 1804 are not concentric, but are offset. As illustrated in Figure 18B, valve block 1806 is fastened to reservoir 1816, and inlet 1810 and elastic membrane 1804 are aligned on one end of valve block 1806. Outlet 1811 and hydrophobic membrane 1802 are aligned on the other end of valve block 1806. Other than the relative position of their components, the hydrophobic check valves 1800 of Figures 18A and 18B function the same.

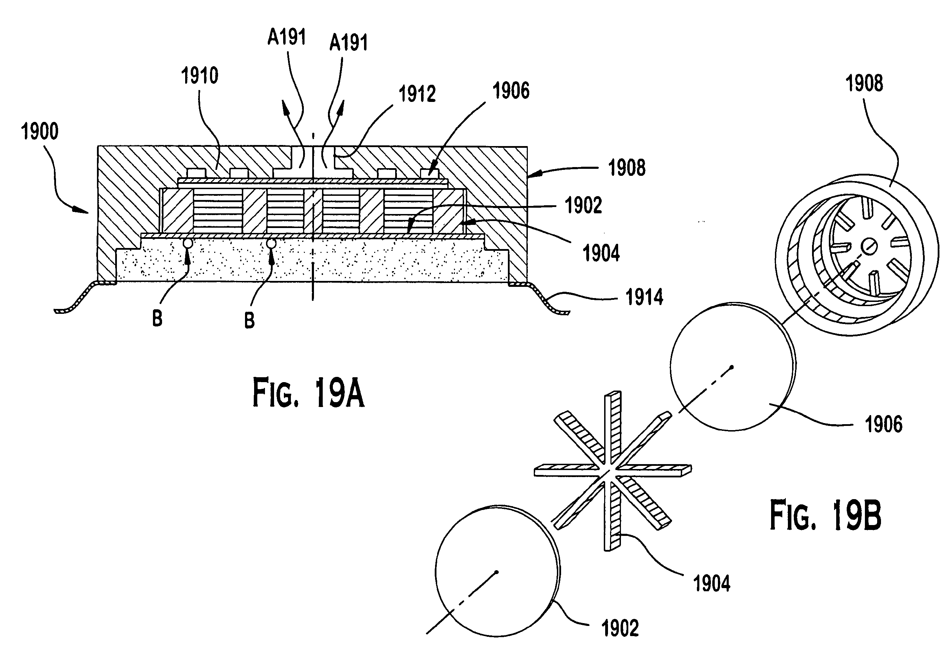

Figures 19A-19B are perspective and cross sectional views of a hydrophilic/hydrophobic check valve 1900 . Hydrophilic/hydrophobic check valve 1900 can be used to vent air during the filling of a reservoir, and to prevent air from flowing into a reservoir when liquids are drawn from the reservoir. Hydrophilic/hydrophobic check valve 1900 prevents direct contact, in the reservoir, between a hydrophobic membrane and the contents of the reservoir. This is particularly beneficial when the reservoir contains pharmaceutical solutions, such as insulin, since aggregates can form when pharmaceutical solutions are in direct contact with hydrophobic surfaces. Hydrophilic/hydrophobic check valve 1900 comprises hydrophilic membrane 1902, spacer 1904, hydrophobic membrane 1906, and valve block 1908. Hydrophobic membrane 1906 and hydrophilic membrane 1902 are hermetically sealed around their perimeters to valve block 1908, while spacer 1904 is positioned between and supports hydrophobic membrane 1906 and hydrophilic membrane 1902. Valve block 1908 includes outlet 1912, and can be attached to reservoir 1914. Spacer 1904 and valve block 1908 are typically made out of rigid materials, such as plastic or metal. Hydrophobic membrane 1906 and hydrophilic membrane 1902 can be made using a variety of materials, as long as hydrophobic membrane 1906 repels water and hydrophilic membrane 1902 attracts water. As reservoir 1914 is filled, air moves toward and passes through hydrophilic membrane 1902. Eventually, all of the air passes through hydrophilic membrane 1902, and is followed by liquid. Liquid passes through hydrophilic membrane 1902, and fills the cavity between hydrophilic membrane 1902 and hydrophobic membrane 1906, pushing air through hydrophobic membrane 1906. Eventually, the cavity between hydrophilic membrane 1902 and hydrophobic membrane 1906 completely fills with liquid, but the liquid does not pass through hydrophobic membrane 1906. It is essentially trapped in the cavity between hydrophilic membrane 1902 and hydrophobic membrane 1906. Once hydrophilic membrane 1902 fills with liquid, air will no longer pass, as indicated by bubbles B in Figure 19A. In addition, as liquid is pumped from reservoir 1914, air cannot pass into reservoir 1914 because it won't pass through hydrophilic membrane 1902 once it is wet. Figure 19B illustrates hydrophilic membrane 1902, spacer 1904, hydrophobic membrane 1906, and valve block 1908, before they've been assembled and attached to a reservoir. Hydrophilic/hydrophobic check valve 1900 is particularly useful when incorporated in the pump engines and systems described and illustrated herein. For example, hydrophilic/hydrophobic check valve 1900 can be attached to a reservoir, allowing air to escape when the reservoir is being filled, but preventing air from being drawn into the reservoir as fluid passes from the reservoir to the pump engine.

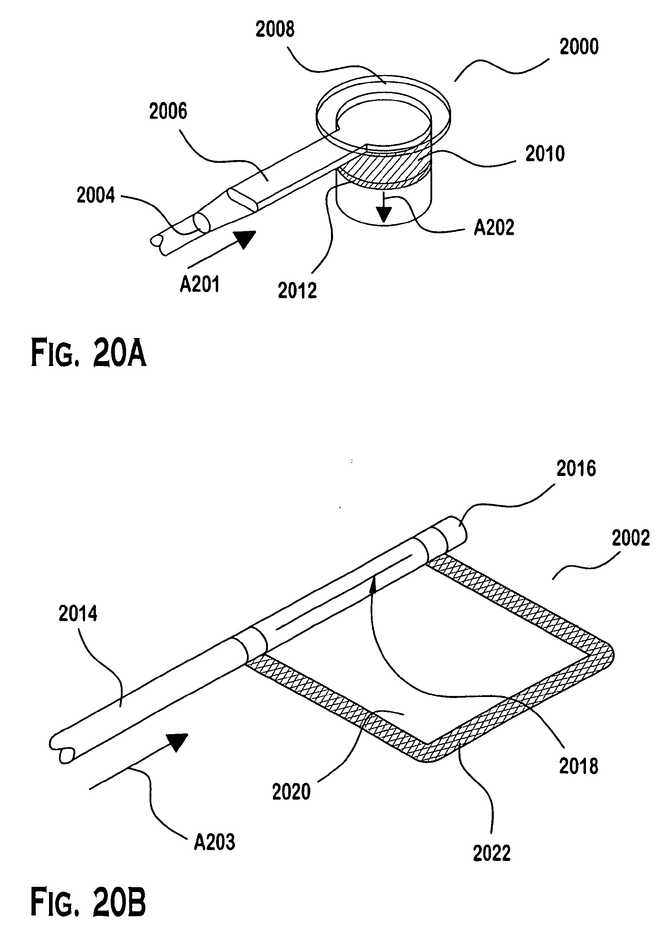

Figures 20A-20B are perspective views of reservoirs 2000 and 2002 . Reservoirs 2000 and 2002 eliminate undesirable air pockets while filling, and are particularly useful when incorporated in the pump engines and systems described and illustrated herein. As illustrated in Figure 20A, reservoir 2000 comprises first inlet channel portion 2004, second inlet channel portion 2006, hydrophobic vent 2008, reservoir chamber 2010, and reservoir piston 2012. First channel portion 2004 transitions in cross section to second channel portion 2006 before reaching hydrophobic vent 2008. Hydrophobic vent 2008 can be made using a variety of materials, such as hydrophobic membranes. Reservoir chamber 2010, first channel portion 2004, and second channel portion 2006 are typically made out of a rigid material, such as plastic or metal, while reservoir piston is typically made out of a semi-rigid material such as an elastomer or other plastic. When filling reservoir 2000, liquid is injected through first channel portion 2004 and second channel portion 2006 (as indicated by arrow A201), and reaches hydrophobic vent 2008. Air passes through hydrophobic vent 2008, and, as pressure increases, reservoir piston 2012 moves down, enlarging reservoir chamber 2010 and filling it with liquid. As illustrated in Figure 20B, reservoir 2002 comprises inlet 2014, hydrophobic vent 2016, burst slit 2018, and reservoir chamber 2020. Hydrophobic vent 2016 can be made using a variety of materials, such as hydrophobic porous plugs or discs. Reservoir chamber 2020 is typically made out of a thin flexible film, such as polyethylene, polyester, or vinyl, and includes a heat seal 2022 around its edge. Inlet 2014 is typically made out of a rigid material, such as plastic or metal, and includes burst slit 2018 that allows flow into reservoir chamber 2020 when it is opened. When filling reservoir 2002, liquid is injected through inlet 2014 (as indicated by arrow A203), and reaches hydrophobic vent 2016. Air passes through hydrophobic vent 2016, and, as pressure increases, burst slit 2018 opens, allowing reservoir chamber 2020 to completely fill with liquid.

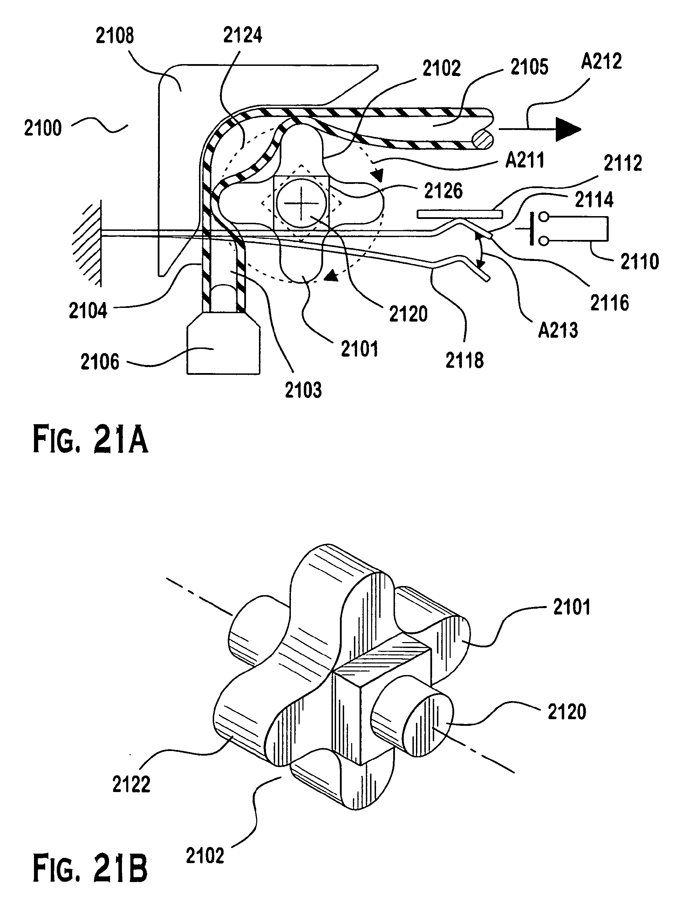

Figures 21A-21B are cross sectional and perspective views of a peristaltic fluid counter 2100 . Peristaltic fluid counter 2100 measures the volume of fluid that flows through it, and is particularly useful when incorporated into the pump engines and systems described and illustrated herein. Peristaltic fluid counter 2100 can be placed adjacent to a reservoir and used to measure the amount of liquid loaded into the reservoir, or it can placed adjacent to the inlet or outlet of a pump engine to monitor the flow of liquid into or out of a pump engine. The arrangement illustrated in Figures 21A-21B is particularly useful in monitoring the volume of liquid that enters a reservoir during the filling of the reservoir. As illustrated in Figure 21A, peristaltic fluid counter 2100 comprises rotor 2102, flexible tube 2104, septum 2106, constraining feature 2108, and switch 2110. Rotor 2102 includes wipers 2101, shaft 2120, and cam 2122. As rotor 2102 rotates about shaft 2120, cam 2122 imparts periodic motion to lever 2114, making and breaking electrical contact with plate 2112. Rotor 2102 can be made out of a variety of materials, both rigid and not rigid, including plastics and metals. Rotor 2102 may be made out of a lubricious polymer, such as Delrin or Teflon, to reduce friction between rotor 2102 and flexible tube 2104. Flexible tube 2104 includes inlet 2101 and outlet 2105, and is elastic. In the arrangement illustrated in Figure 21A, inlet 2101 is connected to a source of liquid, such as a vial, and outlet 2105 is connected to a reservoir. Flexible tube 2104 can be made out of a variety of materials, including elastomers and plasticized PVC. Septum 2106 is connected to inlet 2101, and allows a source of liquid (such as a vial) to be connected to peristaltic fluid counter 2100. Septum 2106 is typically made out of an elastomer, and is self sealing. Constraining feature 2108 supports flexible tube 2104, allowing flexible tube 2104 to expand and contract as fluid flows through it. Constraining feature 2108 is typically made out of a rigid material, such as plastic or metal. Switch 2110 determines when lever 2114 makes and breaks electrical contact with plate 2112, as rotor 2102 rotates about shaft 2120 when fluid flows through flexible tube 2104. As fluid flows through septum 2106, into inlet 2103, and through outlet 2105 (as indicated by 2102 to rotate in the direction indicated by arrow A211. As rotor 2102 rotates, cam 2122 moves lever 2116 up and down (as indicated by arrow A213), making and breaking electrical contact between lever 2116 and plate 2112. Electrical contact between lever 2116 and plate 2112 can be monitored using switch 2110, and can be correlated to volumetric flow through flexible tube 2104. Although in this example peristaltic fluid counter 2100 has been connected to a reservoir, peristaltic fluid counter can be used wherever flow occurs in any of the pump engines and systems described previously.

While the invention has been described in terms of particular variations and illustrative figures, those of ordinary skill in the art will recognize that the invention is not limited to the variations or figures described. In addition, where methods and steps described above indicate certain events occurring in certain order, those of ordinary skill in the art will recognize that the ordering of certain steps may be modified and that such modifications are in accordance with the variations of the invention. Additionally, certain of the steps may be performed concurrently in a parallel process when possible, as well as performed sequentially as described above. Therefore, to the extent there are variations of the invention, which are within the spirit of the disclosure or equivalent to the inventions found in the claims, it is the intent that this patent will cover those variations as well.

## Claims

1. A volumetric micropump, comprising:
a reservoir for containing a quantity of fluid; and
a pump (300, 400, 700, 1600) for withdrawing fluid from the reservoir and delivering fluid to an outlet (308, 408, 708, 1608),
wherein the pump (300, 400, 700, 1600) comprises a pumping chamber (314, 414, 714, 1614) having an inlet (306, 406, 706, 1606) in fluid communication with the reservoir; and
a piston (200, 304, 404, 704, 1604) having a portion of larger cross-sectional area and a portion of smaller cross-sectional area passing through a wall of the pumping chamber (314, 414, 714, 1614);
**characterized in that**:
the reservoir is a flexible reservoir;
the inlet (306, 406, 706, 1606) has a check valve (310, 410, 710, 1610) to inhibit the entry of fluid into the flexible reservoir from the pumping chamber (314, 414, 714, 1614);
the pumping chamber (314, 414, 714, 1614) has an outlet (308, 408, 708, 1608) for fluid communication with a medical infusion device and having a check valve (312, 412, 712, 1612) disposed therein for inhibiting the entry of fluid into the pumping chamber (314, 414, 714, 1614); and
the portion of the piston (200, 304, 404, 704, 1604) of larger cross-sectional area also passes through a wall of the pumping chamber (314, 414, 714, 1614), such that movement of the piston (200, 304, 404, 704, 1604) causes the amount of one of the portions of the piston (200, 304, 404, 704, 1604) disposed within the pumping chamber (314, 414, 714, 1614) to increase and the amount of the other to decrease, to control the volume of the fluid withdrawn from the flexible reservoir and ejected via the outlet (308, 408, 708, 1608) to the medical infusion device.

## Patentansprüche

1. Volumetrische Mikropumpe, umfassend:
einen Vorratsbehälter zum Aufnehmen einer Menge von Fluid; und
eine Pumpe (300, 400, 700, 1600) zum Abziehen von Fluid aus dem Vorratsbehälter und Zuführen des Fluids zu einem Auslass (308, 408, 708, 1608),
wobei die Pumpe (300, 400, 700, 1600) eine Pumpkammer (314, 414, 714, 1614) umfasst, die einen Einlass (306, 406, 706, 1606) in Fluidverbindung mit dem Vorratsbehälter hat; und
einen Kolben (200, 304, 404, 704, 1604), der einen Teil mit einer größeren Querschnittsfläche und einen Teil mit einer kleineren Querschnittsfläche hat, der durch eine Wand der Pumpkammer (314, 414, 714, 1614) läuft:
**dadurch gekennzeichnet, dass**:
der Vorratsbehälter ein flexibler Vorratsbehälter ist;
der Einlass (306, 406, 706, 1606) ein Rückschlagventil (310, 410, 710, 1610) zum Verhindern des Eintritts von Fluid aus der Pumpkammer (314, 414, 714, 1614) in den flexiblen Vorratsbehälter hat;
die Pumpkammer (314, 414, 714, 1614) einen Auslass (308, 408, 708, 1608) zur Fluidverbindung mit einer medizinischen Infusionsvorrichtung hat und ein Rückschlagventil (312, 412, 712, 1612) hat, das darin zum Verhindern des Eintritts von Fluid in die Pumpkammer (314, 414, 714, 1614) angeordnet ist; und
der Teil des Kolbens (200, 304, 404, 704, 1604) mit einer größeren Querschnittsfläche auch durch eine Wand der Pumpkammer (314, 414, 714, 1614) derart läuft, dass die Bewegung des Kolbens (200, 304, 404, 704, 1604) bewirkt, dass die Menge des einen der Teile des Kolbens (200, 304, 404, 704, 1604), der innerhalb der Pumpkammer (314, 414, 714, 1614) angeordnet ist, sich vergrößert und die Menge des anderen sich verringert, um das Volumen des Fluids, das aus dem flexiblen Vorratsbehälter abgezogen und über den Auslass (308, 408, 708, 1608) zur medizinischen Infusionsvorrichtung ausgestoßen ist, zu steuern.

## Revendications

1. Micropompe volumétrique, comprenant :
un réservoir pour contenir une quantité de fluide ; et
une pompe (300, 400, 700, 1600) pour extraire du fluide du réservoir et distribuer du fluide vers une sortie (308, 408, 708, 1608),
la pompe (300, 400, 700, 1600) comprenant une chambre de pompage (314, 414, 714, 1614) ayant une entrée (306, 406, 706, 1606) en communication fluidique avec le réservoir ; et
un piston (200, 304, 404, 704, 1604) ayant une portion de plus grande surface en section transversale et une portion de plus petite surface en section transversale passant à travers une paroi de la chambre de pompage (314, 414, 714, 1614) ;
**caractérisée en ce que**
le réservoir est un réservoir flexible ;
l'entrée (306, 406, 706, 1606) a un clapet anti-retour (310, 410, 710, 1610) pour empêcher l'entrée de fluide dans le réservoir flexible depuis la chambre de pompage (314, 414, 714, 1614) ;
la chambre de pompage (314, 414, 714, 1614) a une sortie (308, 408, 708, 1608) pour la communication fluidique avec un dispositif d'infusion médical et ayant un clapet anti-retour (312, 412, 712, 1612) disposé dans celle-ci pour empêcher l'entrée de fluide dans la chambre de pompage (314, 414, 714, 1614) ; et
la portion du piston (200, 304, 404, 704, 1604) de plus grande surface en section transversale passe aussi travers une paroi de la chambre de pompage (314, 414, 714, 1614), de telle sorte que le mouvement du piston (200, 304, 404, 704, 1604) provoque l'augmentation de la quantité de l'une des portions du piston (200, 304, 404, 704, 1604) disposées dans la chambre de pompage (314, 414, 714, 1614) et la diminution de la quantité de l'autre, afin de réguler le volume du fluide extrait du réservoir flexible et éjecté par la sortie (308, 408, 708, 1608) vers le dispositif d'infusion médical.
